(19) Europäisches Patentamt
European Patent Office
Office européen des brevets

(11) EP 1 222 921 B1

(12) EUROPEAN PATENT SPECIFICATION

(45) Date of publication and mention of the grant of the patent:
**03.11.2004 Bulletin 2004/45**

(51) Int Cl.[7]: **A61K 31/426**, A61P 31/12, A61P 33/00

(21) Application number: **02002887.4**

(22) Date of filing: **06.05.1998**

(54) **Pharmaceutical compositions of tizoxanide and/or nitazoxanide**

Tizoxanide und/oder Nitazoxanide enthaltende pharmazeutische Zubereitungen

Compositions pharmaceutiques contenant du tizoxanide et/ou du nitazoxanide

(84) Designated Contracting States:
**AT BE CH DE DK ES FI FR GB GR IE IT LI LU MC NL PT SE**

(30) Priority: **07.05.1997 US 852447**
**03.07.1997 US 887810**
**03.07.1997 US 887809**

(43) Date of publication of application:
**17.07.2002 Bulletin 2002/29**

(62) Document number(s) of the earlier application(s) in accordance with Art. 76 EPC:
**98920285.8 / 1 005 342**

(73) Proprietor: **Romark Laboratories, L.C.**
**Tampa, FL 33607 (US)**

(72) Inventor: **Rossignol, Jean-Francois**
**St Petersburg, FL 33710 (US)**

(74) Representative: **Winkler, Andreas, Dr.**
**FORRESTER & BOEHMERT**
**Pettenkoferstrasse 20-22**
**80336 München (DE)**

(56) References cited:
**US-A- 5 387 598**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## BACKGROUND OF THE INVENTION

### Field of the Invention

The present invention relates to a pharmaceutical composition containing as active agent at least one compound selected among the group consisting of formula (I)

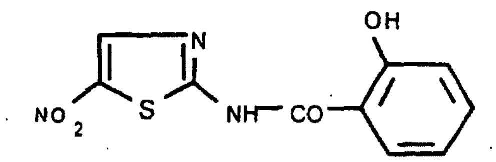

(I)

and formula (II)

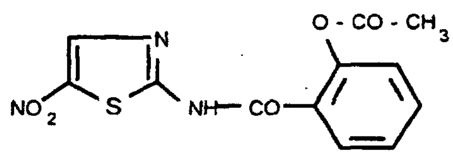

(II),

and further containing at least one pharmaceutically aceptable acid.

The active agent is preferably in the form of particles having a particle size smaller than 200 µm and a mean particle size of greater than 10 µm.

The invention, in particular, relates to pharmaceutical compositions stabilized with at least one pharmaceutically acceptable acid.

The pharmaceutical compositions are particularly useful for treatment of opportunistic infections in persons with compromised or suppressed immune systems, and in treatment of infections of trematodes.

### Description of the Related Art

There is an urgent need for the development of methods for treatment of a number of parasitic and bacterial infections in humans with compromised immune systems (AIDS, cancer patients, elderly, aging, organ transplant patients on immunosuppressive drugs). Another area of concern is trematode infections, particularly in tropical climates. There is thus a need for a pharmaceutical composition which can be tolerated by even immunocompromised humans, and which is storage stable even in tropical environments.

More specifically, *Toxoplasma gondii* is a protozoan and is among the most prevalent causes of latent infection of the central nervous system throughout the world. Many healthy people are infected with the parasite, but usually the immune system keeps the organism under control. *T. gondii* is the most common opportunistic pathogen of the brain in AIDS patients. At present, toxoplasmosis is becoming an increasing problem not only because of AIDS, but also because of wider use of immunosuppressive drugs (e.g., as administered to organ-transplant patients). Toxoplasmosis is usually treated with a combination of pyrimethamine and sulfadiazine. While the drugs are effective, they do not kill cysts of the parasite, so the treatment must be continued as a maintenance dose Toxicity often forces discontinuation of the drug, particularly in the immunosuppressed, and relapses result. The statistics are not good, with reported death rates of about 70 percent in immunodeficient patients and median survival of four months.

Cryptosporidiosis is caused by the microscopic protozoan parasite *Cryptosporidium parvum.* In persons with normal immune functions, the diarrhea caused by *C. parvum* may be intense and prolonged, but is self-limiting. In AIDS patients, cryptosporidial diarrhea is often life-threatening. It is estimated that 15-20 percent of AIDS patients suffer from this condition. Up to now, there has been no consistently effective or approved therapy for cryptosporidiosis.

The most frequently identified pathogen in AIDS patients is *Enterocytozoon bieneusi*, a microsporidian parasite, which was found in nearly one-quarter of the patients. It now appears that this tiny parasite may turn out to be the cause of a large proportion of the many unexplained cases of malabsorption, diarrhea and wasting seen in HIV-ill patients. There is no known effective treatment as yet.

Several other species of microsporidia infect HIV-positive patients, including *Encephalitozoon hellem* and *cuniculi,* and a new species designated *Septata intestinalis.* A recent report suggests that disseminated microsporidia infections are increasing in significance.

Infection with the parasite *Isospora belli* is clinically indistinguishable from cryptosporidiosis. More common in tropical climates, *I. belli* has been reported in less than 1% of patients in the U.S., although its actual incidence is probably higher.

*Pneumocystis carinii* has generally been classified as a protozoan parasite; some studies indicate it may be a fungus, with which it shares certain genetic sequences. *P. carinii* usually infects the lungs (Pneumocystis Carinii Pneumonia (PCP)). Therapy is reported to be successful in about 40-60% of patients, with problems including drug toxicity particularly in immunocompromised patients. Among the many serious manifestations of human immunodeficiency virus (HIV) infection in children, PCP stands out because of its high incidence, unique age distribution, and frequent mortality. PCP is the most common serious opportunistic infection in children with HIV infection; the incidence of PCP among HIV-infected infants not receiving prophylaxis is estimated to be at least 12% in the first year of life. Many children die shortly alter PCP develops.

Mycobacterium Avium Complex (MAC) refers to infections by a family of very similar mycobacterial organisms, *Mycobacterium avium* and *M. intracellulare.* When MAC occurs in non-immunocompromised people, it is usually in the form of infection in the respiratory tract. In patients with AIDS, MAC is frequently disseminated (disseminated MAC or DMAC), and almost any organ system can be involved. In a recent study, MAC bacterial was found in 43% of patients who survived for 2 years after an AIDS diagnosis. No standard therapy has been established for disseminated MAC. Combinations of drugs are usually prescribed and, if successful, require that treatment be continued for life. A more effective treatment is urgently needed.

The HIV-infected are particularly susceptible to infection by *Mycobacterium tuberculosis,* and the course of the disease is accelerated. While extrapulmonary tuberculosis is unusual in non-HIV-infected patients, it frequently occurs in HIV-positive people. The CDC has released guidelines for the treatment of TB which address the growing prevalence of multi-drug resistant TB (MDR-TB). Mortality among AIDS patients with MDR-TB is very high (approximately 80%) and the disease progression is extremely rapid.

Accordingly, there is an urgent need for the development of a method of treatment of these infections so prevalent in, and threatening to, humans and animals.

There is also a need for a broad-acting drug for simplification of treatment of trematode infections. Presently, it is necessary to diagnose the specific trematode pathogen, and then to prescribe the drug therapy specific for that trematode. Many lesser developed countries are not equipped to diagnose a the specific trematode. Development of a broad acting drug would eliminate the requirement for diagnosis.

*Schistosoma mansoni,* the blood fluke, is the causative agent of Schistosomiasis, the second most important tropical parasitic disease of man (after Malaria), and the most important trematode infection of man. *Schistosoma haematobium* is another important species infecting man. Over 200 million individuals suffer from schistosomiasis world wide, including several hundred thousand people in the United States.

*Fasciola hepatica,* the common liver fluke, is primarily a disease of sheep, but humans are an accidental host. The parasite manages to survive in the presence of a vigorous host immune response. Bithionol has been suggested for treatment, but is not approved for use in the United States.

There is further thus a need for a pharmaceutical composition which is storage stable even in tropical environments, and which is broad acting against trematodes.

## Summary of the Invention

It has now been observed in animal studies and in human clinical studies that the efficacy of a treatment, using the compounds of formula (I) and (II) is dependent upon the particle size of the active drug substance and the stability of the compounds.

The pharmaceutical compositions described are suitable for treating human and animal trematode infections caused by *Schistosoma* such as *Schistosoma mansoni, Schistosoma haematobium, Schistosoma mekongi, Schistosoma japonicum, Schistosoma intercalatum; Fasciola* such as *Fasciola hepatica* and *Fasciola gigantica, Fasciolopsis biski;* and *Dicrocoelium dendriticum, Heterophyes heterophyes,* and *Metagonimus yokogawa.*

The pharmaceutical compositions are also effective for treatment in the immuno-compromised of opportunistic infections of *Cryptosporidium parvum, Isospora belli, Enterocytzoon bieneusi, Encephalitozoon intestinalis, Mycobacterium tuberculosis, Mycobacterium avium intracellulare, Pneumocystis carinii,* and *Toxoplasma gondii.*

The pharmaceutical composition may be in a form suitable for oral administration, as a solid dosage form, a liquid suspension, or a paste.

## BRIEF DESCRIPTION OF THE DRAWINGS

For a fuller understanding of the nature and objects of the present invention reference should be made by the following detailed description taken in with the accompanying drawings in which:

Fig. 1 shows percent inhibition and host cell viability of nitazoxanide against *E. intestinalis.*
Fig. 2 shows percent inhibition and host cell viability of nitazoxanide against *V. corneae.*
Fig. 3 shows percent inhibition and host cell viability of albendazole against *E. intestinalis.*
Fig. 4 shows percent inhibition and host cell viability of albendazole against *V. corneae.*
Figs. 5 and 6 show a plot of OD values obtained for each *T. gondii* culture well, *vs* the concentration of the drug in the culture.
Fig. 7 is a chart based upon the assay for nitazoxanide effectiveness against mycobacteria growing in a liquid broth.
Fig. 8 shows the percentage of active particles having a size smaller than Ø µm.

## Detailed Description of the Invention

The invention relates to pharmaceutical compositions containing as active agent at least one compound selected from the group consisting of:

compound of formula (I)

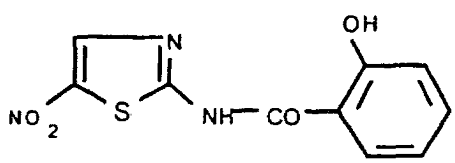

(I)

and compound of formula (II)

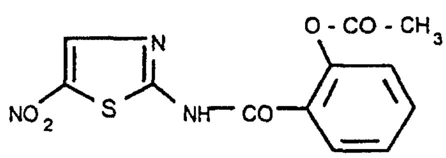

(II),

and further containing a stability improving amount of a pharmaceutically acceptable acid.

The invention further relates to a pharmaceutical composition containing as active agent

a compound of formula II

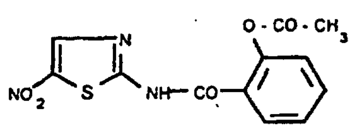

and a pharmaceutically acceptable acid in an amount sufficient to render the pH of said pharmaceutical composition between 2 and 6 when said pharmaceutical composition is contacted with water, wherein said active agent is in the form of solid particles, and wherein the ratio of the weight of the pharmaceutically acceptable acid to the weight of said solid particles is between 0.01 and 0.5.

Examples of such acids are: citric acid, glutamic acid, succinic acid, ethanesulfonic acid, acetic acid, tartaric acid, ascorbic acid, methanesulfonic acid, fumaric acid, adipic acid, malic acid and mixtures thereof. Citric acid is very appropriate. The presence of said acid improves the stability of the active agent or agents.

The ratio of the weight of pharmaceutically acceptable acid to the weight of said active solid particles is advantageously between 0.03 and 0.2. Advantageously, the amount of acid is sufficient for adjusting the pH of the suspension between 2 and 6, preferably between 3 and 5, most preferably between 3.5 and 4.5.

Techniques for preparation of, and preferred examples of, solid and liquid dosage forms of the pharmaceutical composition are disclosed in WO/95/28393, the disclosure of which is incorporated herein by reference. The compositions contain advantageously a wetting agent and possibly a starch derivative such as those disclosed in US Patent 5,578,621, the content of which is incorporated herein by reference for disclosing possible wetting agents and starch derivatives. The wetting agent as described in US 5,578,621 serves as a dispersing agent.

Such pharmaceutical compositions, either as solid or liquid dosage forms or as pastes or ointments, can optionally contain additional active agents such as antibiotics, antiviral agents or proton pump inhibitors. While it is not advantageous, it is also possible that such pharmaceutical formulations contain active solid particles of compound of formula (I) and/or compound of formula (II) which are larger than 200 μm.

The compositions can contain excipients known as such for the purpose of preparing forms suitable for oral administration.

Advantageously, in order to have excellent efficacy against a broad spectrum of parasites, bacteria, fungi and viruses, the distribution factor of said active solid particles is between 0.8 and 2, preferably between 1.1 and 1.9, most preferably greater than 1.5, said distribution factor being calculated by the following formula:

$$F_{90\%} = (Ø_{90\%} - Ø_{10\%})/((Ø_{90\%} + Ø_{10\%})/2)$$

in which

- $F_{90\%}$ is the distribution factor at 90%;
- $Ø_{90\%}$ is the maximum particle size of the fraction of particles corresponding to 90% of said active solid particles, and
- $Ø_{10\%}$ is the maximum particle size of the fraction of particles corresponding to 10% of said active solid particles.

According to a specific embodiment of the invention, particles of a compound of formula (I) and/or (II) are prepared by the methods described hereabove and are then milled so that less than 10% of said active particles are larger than 100 μm, less than 50% of said particles are larger than 50 μm and less than 10% of said active particles are smaller than 5 μm in size, the mean particle size being between 20 and 50 μm. Said active particles are then granulated using a mixture containing active solid particles and at least one granulating agent. Examples of granulating agent are: polyvinylpyrrolidone, water, alcohol, sucrose hydroxyl cellulose and mixture thereof. Advantageously, at least one pharmaceutically acceptable acid is added during the granulation process.

The invention also relates to solid dosage forms containing a composition of the invention such as tablets, dispersible tablets, coated tablets, matrixes, etc. The dosage form of the invention contains, for example:

- solid active particles with a particle size smaller than 200 μm, less than 10% of said particles having a size larger than 100 μm, less than 50% of said particles having a size larger than 50 μm and less than 10% of said particles having a size less than 5 μm, the mean particle size being between 20 and 50 μm.
- at least one granulating agent;
- at least one wetting agent;
- at least one starch derivative, and
- at least one pharmaceutically acceptable acid which is preferably added during the granulation process.

Liquid dosage forms such as aqueous suspensions of the invention contain, for example:

- as active agent, solid particles containing a compound of formula (I) and/or a compound of formula (II) having a particle size smaller than 200 μm, less than 10% of said particles having a size larger than 100 μm, less than 50% of said particles having a size larger than 50 μm and less than 10% of said particles having a size less than 5 μm, and
- at least one granulating agent;
- at least one wetting agent;
- at least one pharmaceutically acceptable acid, the pH of the suspension being between 2 and 6, preferably between 3 and 5, most preferably between 3.5 and 4.5;
- at least one thickener, for example a Xanthan gum, aguar gum, crystalline cellulose, carruba gum, carboxymethylcellulose or a mixture thereof.

Paste or ointment forms of the invention suitable for oral administration contain, for example:

- as active agent, solid particles containing a compound of formula (I) and/or a compound of formula (II) having a particle size smaller than 200 μm, less than 10% of said particles having a size larger than 100 μm, less than 50%

of said particles having a size larger than 50 µm and less than 10% of said particles having a size less than 5 µm, and

- at least one wetting agent;
- at least one pharmaceutically acceptable acid, the pH of the suspension being between 2 and 6, preferably between 3 and 5, most preferably between 3.5 and 4.5;
- at least one thickener, for example a Xanthan gum, aguar gum, crystalline cellulose, carruba gum, carboxymethylcellulose or a mixture thereof.

Paste or ointment forms for topical or intravaginal application contain, for example:

- as active agent, solid particles containing a compound of formula (I) and/or a compound of formula (II) having a particle size smaller than 200 µm, less than 10% of said particles having a size larger than 100 µm, less than 50% of said particles having a size larger than 50 µm and less than 10% of said particles having a size less than 5 µm, and
- at least one wetting agent;
- at least one pharmaceutically acceptable acid, the pH of the suspension being between 2 and 6, preferably between 3 and 5, most preferably between 3.5 and 4.5;
- cetylic alcohol and/or glyceride derivatives and/or propyleneglycol;
- at least one thickener, for example a Xanthan gum, aguar gum, crystalline cellulose, carruba gum, carboxymethylcellulose or a mixture thereof.

A method for treatment of infections comprises administration of a pharmaceutical composition comprising, as active agent, at least one compound selected from the group consisting of desacetyl-nitazoxanide of formula (I):

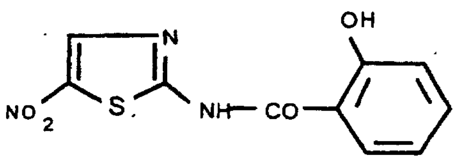

(I)

and nitazoxanide of formula (II):

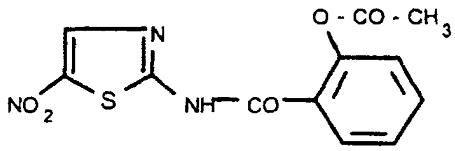

(II).

Nitazoxanide (NTZ), the compound of formula (II), is the generic name for 2-(acetolyloxy)-N-(5-nitro 2-thiazoly) benzamide, a compound first synthesized by Rossignol and Cavier in 1975. 2 mg of nitazoxanide can be dissolved in 1 ml DMSO. Nitazoxanide is easily absorbed orally.

Until now there has been no evidence that the compounds of formula (I) and/or (II) could be broadly effective against trematode infections, or that they would be sufficiently nontoxic to be tolerated by even immuno-compromised humans.

The preparation and certain uses of nitazoxanide is disclosed in US Patent 3,950,351, as well as in publications by the present inventor. Desacetyl-nitazoxanide, the compound of formula (I), is sometimes referred to as tizoxanide or d-NTZ, and is a metabolite of nitazoxanide.

In WO 95/28393, the present inventor disclosed a method for the manufacture of pure compound of formula (II), as well as the use of the composition containing a mixture of compounds of formula (I) and (II).

It has now been observed that solid particles of compound of formula (I), compound of formula (II), or mixtures thereof having a particle size between 170 and 520 µm (mean particle size = 352 µm) have very limited efficacy when administered orally to animals or humans. The efficacy of such particles is inferior to existing pharmaceutical products and therefore unacceptable for regulatory or commercial purposes.

It has also been observed in dogs that the oral administration of a single dose of 50 milligrams per kilogram of solid particles of compound of formula (I) and compound of formula (II) having a particle size smaller than 5 µm, caused severe adverse reactions in the animals.

It has now been discovered that in order to have an effective and safe treatment of infections caused by parasites, bacteria, fungi and viruses in humans and animals, the pharmaceutical composition, either a solid dosage form or an aqueous suspension, must contain the effective dose of the active agent in the form of solid particles having a particle size smaller than 200 µm and containing compound of formula (I) and/or compound of formula (II), the mean particle size of the active solid particles being greater than 10 µm.

The presence of a high content of particles of active agent having a size larger than 200 µm with respect to the content of particles having a size between 5 and 200 µm significantly reduces the chemotherapeutic activity of the compounds. Preferably, the pharmaceutical compositions of the invention do not contain more than 5% by weight of active solid particles having a size larger than 200 µm. Most preferably, the pharmaceutical compositions of the invention contain substantially no active solid particles having a size larger than 200 µm.

The presence of a high content of particles of active agent having a size less than 5 µm with respect to the content of particles having a size between 5 and 200 µm can produce adverse effects in animals or in humans. In addition, it has been observed that particles having a size less than 5 µm are more rapidly absorbed from the gastro-intestinal tract into the bloodstream, and therefore are not as effective against parasites, bacteria, fungi and viruses which commonly live within the gastro-intestinal tract of animals and humans.

The skilled scientist could not predict that the particle size of compound of formula (I) and compound of formula (II) would have such a significant impact upon its antimicrobial activity in animals and in humans. For example, in studies conducted by the Inventor, anti-parasitic compounds such as albendazole, mebendazole, niclosamide, praziquantel and metronidazole have not demonstrated such a marked difference in anti-parasitic activity in animals or humans which was dependent upon their particle size. In addition, a skilled scientist could not predict that particle sizes of compound of formula (I) and compound of formula (II) would have such an adverse impact upon the ability of animals or humans to tolerate the administration of said active agent.

The compound(s) of formula (I) and (II) may be administered in either a solid dosage form or an aqueous suspension, and it is preferred that the pharmaceutical composition contain the effective dose of the active agent in the form of solid particles of formula (I) and/or (II) having a particle size smaller than 200 µm, the mean particle size of said active solid particles being greater than 10 µm as determined by a Coulter® Counter LS 100. This equipment uses laser light at 750 nm to size particles from 0.4 to 900 µm in diameter by light diffraction. The samples are measured in water with a small amount of Triton X-100 in order to increase the wettability and deflocculate the powder.

Advantageously, the mean particle size of said active solid particles is between 10 and 100 µm, preferably between 20 and 50 µm. Examples of preferred compositions are:

- a composition for which less than 10% by weight of said active solid particles has a particle size larger than 100 µm;
- a composition for which at least 50% by weight of said active solid particles has a particle size smaller than 50 µm.

Advantageously, the mean particle size of said active solid particles is between 10 and 100 µm, preferably between 20 and 50 µm. In accordance with a preferred embodiment of the composition, less than 10% of said active solid particles has a particle size smaller than 5 µm.

The active agent or agents used in the solid dosage form or suspension is advantageously a mixture of solid particles of compounds of formula (I) and of formula (II) with a particle size smaller than 200 µm, the weight content of compound of formula (I) with respect to the weight of compounds of formula (I) and of formula (II) of said mixture being comprised between 0.5 and 20%, preferably between 0.5 and 10%.

**Description of Preparation of Pharmaceutical Compositions**

Dry pure compound of formula (I) and dry pure compound of formula (II) were submitted to grinding and sized by means of a mesh screen.

After grinding, the particles of compound of formula (I), of formula (II) and mixtures thereof had the particle size distribution as given in Fig. 8. Fig. 8 shows the percentage of particles having a size smaller than Ø µm.

From said figure, it appears that:

- less than 10% by weight of the particles had a particle size smaller than approximately 5 µm;
- less than 10% by weight of the particles had a particle size larger than approximately 70 µm;
- the mean particle size is approximately 40 µm;
- the distribution factor of the particles is about 1.73, said distribution factor being calculated by the following formula:

$$F_{90\%} = (Ø_{90\%} - Ø_{10\%})/((Ø_{90\%} + Ø_{10\%})/2)$$

in which

- $F_{90\%}$ is the distribution factor at 90%;
- $Ø_{90\%}$ is the maximum particle size of the fraction of particles corresponding to 90% of said active solid particles, and
- $Ø_{10\%}$ is the maximum particle size of the fraction of particles corresponding to 10% of said active solid particles.

Specific examples of such compositions are disclosed in the following Tables.

Table 1.

| **Example of composition of dispersible tablets for oral administration containing compound of formula (II) and compound of formula (I) as active agents.** | |
|---|---|
| Nitazoxanide (99%) + desacetyl-nitazoxanide (1%) | 200 mg |
| Microcrystalline cellulose | |
| Avicel pH 102 sold by FMC-USA | 116 mg |
| Crospovidone | 25 mg |
| Magnesium stearate | 3 mg |
| Colloidal silicon dioxide | 5 mg |
| Citric acid | 10 mg |
| Strawberry flavor No. 877720 sold by Robertet | 10 mg |
| Sodium saccharinate | 2 mg |

Table 2.

| **Example of composition of coated tablets for oral administration containing compound of formula (II) and compound of formula (I) as active agents.** | |
|---|---|
| Nitazoxanide | 500 mg |
| Maize starch | 60 mg |
| Pregelatinized Maize starch | 70 mg |
| Hydroxypropyl methylcellulose | 5 mg |
| Sucrose | 20 mg |
| Sodium starch glycollate | 30 mg |
| Citric acid | 25 mg |
| Talc | 8 mg |
| Magnesium stearate | 7 mg |

Coatings:

Hot sugar solution or a film coating being sprayed on the tablets or granules containing 500 mg active agent

Table 3.

| **Example of an aqueous suspension for oral administration containing compound of formula (II) and compound of formula (I) as active agents. The pH of the suspension was about 4.1.** | |
|---|---|
| Nitazoxanide (98%) + desacetyl-Nitazoxanide (2%) | 2 g |
| Distilled water | 100 ml |
| Sodium benzoate | 0.2 g |
| Saccharose | 30.5 g |
| Xanthan gum | 0.2 g |
| Microcrystalline cellulose and carboxymethylcellulose sodium | |
| Avicel RC-591 sold by FMC - USA | 0.8 g |
| Citric acid | 0.2 g |
| Dihydrated sodium citrate | 50 mg |
| Strawberry flavor No. 877720 sold by Robertet | 125 mg |

Table 3. (continued)

| Example of an aqueous suspension for oral administration containing compound of formula (II) and compound of formula (I) as active agents. The pH of the suspension was about 4.1. | |
|---|---|
| Red dye No. 33 D and C | 1 mg |

Table 4.

| Example of a paste for oral administration containing compound of formula (II) and compound of formula (I) as active agents. | |
|---|---|
| Nitazoxanide (98%) + desacetyl-Nitazoxanide (2%) | 500 mg |
| Mineral oil | 10 g |
| Brown sugar | 1 g |
| Microcrystalline cellulose and carboxymethylcellulose sodium | |
| Avicel RC-591 sold by FMC | 0.8 g |
| Citric acid | 0.2 g |

Table 5.

| Example of a paste or ointment formulation for intravaginal or topical application, said paste or ointment containing compound of formula (II) and compound of formula (I) as active agents. | |
|---|---|
| Nitazoxanide (98%) + desacetyl-Nitazoxanide (2%) | 8 g |
| Cremaphor A6 | 2 g |
| Cremaphor A25 | 1.5 g |
| Mineral oil | 7 g |
| Luvitol EHO | 7 g |
| Glycerol monoester | 4 g |
| Cetylic alcohol | 3 g |
| Simeticone | 0.5 g |
| Germaben II | 1 g |
| Propyleneglycol | 3.5 g |
| Distilled water | 62.5 g |

The pharmaceutical compositions of the invention are compositions having a broad spectrum of action on parasites, bacteria, fungi and viruses, especially when administered orally.

The efficacy and the safety of the pharmaceutical compositions disclosed hereabove were excellent in animals and in humans. Specifically, in human clinical studies, it has been observed that the efficacy of the pharmaceutical compositions described hereabove are significantly more effective in treating parasitic infections than the same formulations using active compound having particle sizes between 170 and 520 µm (mean particle size = 352 µm), even when the larger sized particles were administered to patients at doses up to three times higher and for longer periods of time. Examples of cure rates obtained are shown below in Table 6.

Table 6.

| **Comparison of results of human clinical studies using compounds of formula (I) and formula (II) having particle sizes ranging from 170 µm to 520 µm (mean = 352 µm) with results obtained using formula (I) and formula (II) having particle sizes ranging from 5 µm to 200 µm (mean = 34 µm).** | | |
|---|---|---|
| **Compound of formula (I) (98%) + Compound of formula (II) (2%)** | | |
| | ***Particle sizes 170 to 520 µm*** | **Particle *sizes 5 to 200 µm*** |
| | ***Dose = 15 to 50 mg/kg/day for 3 to 7 days*** | ***Dose = 15 mg/kg/day for 3 days*** |
| | **No. Cured/Total = % Cure Rate** | **No. Cured/Total = % Cure Rate** |
| **Parasite** | | |
| Blastocystis hominis | 12/27 = 44% | 10/10 = 100% |
| Entamoeba histolytica | 29/47 = 62% | 106/133 = 80% |
| Giardia lamblia | 11/37 = 30% | 50/73 = 68% |
| Ascaris lumbricoides | 3/69 = 4% | 144/179 = 80% |
| Trichuris trichiura | 7/48 = 15% | 58/79 = 73% |

For each of the parasites listed in Table 6, the proportional cure rates were significantly better for the patients treated with active particles between 5 and 200 µm than for those treated with active particles ranging from 170 µm to 520 µm in size, with a statistical significance in each case being $p < 0.02$ (using a standard $X^2$ test). This was the case even though the doses of the larger particle size active agent were usually higher and the duration of treatment was often longer than that administered to the patients receiving pharmaceutical compositions of active agent having particle sizes smaller than 200 µm. There were no serious adverse effects reported for either group of patients.

Results similar to those described above for human studies have also been observed in animal testing.

In addition, the adverse reactions observed in dogs after oral administration of a single dose of 50 milligrams per kilogram of compound of formula (I) and compound of formula (II) have not been observed in extensive studies in animals using compound of formula (I) and compound of formula (II) having a particle size between 5 and 200 µm (mean > 10 µm), even when the same dose or a higher dose of the compounds were administered daily for 90 days or longer.

Moreover, said compositions were stable (even when subjected to temperatures of 40° C and 65% relative humidity for six months or, in the case of liquid suspensions, when suspended in water under these conditions for 3 months) thereby assuring that the active ingredients do not degrade and that the compositions maintain their efficacy for a period of time after their preparation which is suitable for medicinal and commercial purposes.

In the following, efficacy of the pharmaceutical compositions will be demonstrated.

## EXAMPLE I

*CRYPTOSPORIDIUM PARVUM*

In a preliminary clinical trial, 30 AIDS patients with chronic cryptosporidial diarrhea were treated with oral nitazoxanide from 500 to 2000 mg daily. If the diarrhea continued, the patients received an additional four weeks of nitazoxanide, up to 2000 mg a day.

Twenty-eight people completed two or more weeks of therapy and 16 of those were evaluable for a therapeutic response by the eighth week of treatment. In this latter group, 12 persons had a 50 percent or greater reduction in daily bowel movement frequency and 10 individuals had a marked reduction or eradication of the parasite in the stool, with the organism becoming undetectable in four people. Six patients met both clinical and parasitological response criteria for benefit.

Patients who received higher daily doses of drug for longer periods of time were more likely to have a positive response.

An open-label study of nitazoxanide for AIDS-related cryptosporidial diarrhea documented decreased bowel movements among persons taking 500, 1000, 1500, or 2000 mg of the drug daily. Trial participants had a mean CD4+ count of 42 cells/$mm^3$ (range 0-303 cells/$mm^3$), a mean 6.7 bowel movements daily for an average 15 months, *Cryptosporidium parvum* oocysts in stool, and no other apparent enteric pathogens. Almost all participants had failed therapy with azithromycin or paromomycin.

After 23 weeks, 9 of 13 had a complete clinical response (one to three predominantly formed bowel movements daily), and 4 of 13 had a partial clinical response (at least a 50 percent decrease in daily bowel movements or a change in stool consistency so that at least 75 percent were formed). By the end of the study, 8 of 11 had completely eradicated the parasite and the other three had substantial reductions in oocyst levels. There was a trend toward better response with doses of 1000 mg daily or higher and with longer therapy. Two trial participants had urticarial skin rashes; more than 90 percent adhered to the study regimen for more than four weeks.

**EXAMPLE II**

*CRYPTOSPORIDIUM PARVUM*

In vitro Dose Information:

Nitazoxanide was dissolved in sterile dimethylsulfoxide (DMSO) and tested against intact *C. parvum* oocysts infected cell monolayers at concentrations of 100 µg/ml, 10 µg/ml/l µg/ml and 0.1 µg/ml. A second trail was performed which tested nitazoxanide at the additional concentrations of 20, 2, 0.2 and 0.02 µg/ml. These concentrations were achieved by serial dilutions with complete DMEM medium to yield a final DMSO concentration of 0.5%. The medium control also contacted 0.5% DMSO.

The experiment used a cell culture of MDBKF5D2 Cells grown in 7 mm chambers, and as *Cryptosporidium parvum:* GCH1 oocysts, 5 X $10^4$ per well, and was conducted to compare paromomycin (positive control) against nitazoxanide (experimental drug). Materials included Immune Anti-*Cryptosporidium parvum* Sporozoite Rabbit Serum (0.1%) and Fluorescein-Conjugated Goat Anti-Rabbit Antibody (1%).

Toxicity Testing Assay:

200µl of medium containing nitazoxanide at concentrations of 100, 10, 1 and 0.1 µg/ml and the proper controls were introduced into two wells of a 96 well plate containing confluent MDBKF5D2 cell monolayers and two wells without monolayers. The drug was incubated on the monolayers at 37°C and 8% $CO_2$. At 24 hours (trial 1) and 48 hours (trial 2), MTS (Owen's solution) the PMS were added to each well at concentrations of 333 µg/ml and 25 µM respectively. The plate was returned to the incubator in the dark to develop for two hours. At two hours, 100 µl of each supernatant was transferred to a new microtiter plate and read in the ELISA reader at 490nm. Results were recorded and analyzed. Percent toxicity was calculated by subtracting the mean optical density (OD) of the drug supernatants from the mean optical density (OD) of the medium control supernatants (no drug), dividing by the OD of the medium control and multiplying by 100.

$$\frac{\text{OD medium - OD drug}}{\text{OD medium}} \times 100$$

Intact *C. parvum* Oocyst Assay:

5 x 104 *C. parvum* oocysts per well were incubated in nitazoxanide (100, 20, 10, 2, 1, 0.2, 0.1 and 0.02 µg/ml) at 37°C (8% $CO_2$) on confluent MDBKF5D2 cell monolayers. The level of infection in each well was determined and analyzed by an immunofluorescence assay at 24 to 48 hours. Percent Inhibition was calculated by subtracting the mean parasite count/10 field in the drug test wells from the mean parasite count/10 fields in the medium control (no drug), dividing by the medium control count, and then multiplying by 100.

$$\frac{\text{Medium control count - Drug test count}}{\text{Medium control count}} \times 100$$

Results:

Trial 1: 24 hrs.

| Compound | Conc. | Mean (+ SD)* | Percent Toxicity | Percent Inhibition |
|---|---|---|---|---|
| Infected Media | 0 | 983.5(±128.2) | 0 | 0 |
| Paromomycin | 2 mg/ml | 482(±47.1) | 23.8 | 51 |
| NTZ | 100μg/ml | Lost | 88.1 | NA** |
| | 10μg/ml | 55.5(±13.5) | 65.1 | 94.4 |
| | 1μg/ml | 224.5(±28.5) | 8.3 | 77.2 |
| | 0.1μg/ml | 474.5(±29.5) | 19.3 | 51.8 |

* Parasite Count/10 Fields

**Not available due to toxicity

Trial 2: 48 hrs.

| Compound | Conc. | Mean (+ SD)* | Percent Toxicity | Percent Inhibition |
|---|---|---|---|---|
| Infected Media | 0 | 2231.25(+90.03) | 0 | 0 |
| Paromomycin | 2 mg/ml | 580(+33.42) | 40.8 | 74.01 |
| NTZ | 20μg/ml | 68.75(+13.77) | 92.87 | 96.92 |
| | 2μg/ml | 113.75(+21.36) | 24.93 | 94.90 |
| | 0.2μg/ml | 1020(+158.48) | 16.56 | 54.29 |
| | 0.02μg/ml | 1041(+191.46) | 21.23 | 53.33 |

* Parasite Count/10 Fields

Impact of nitazoxanide on the intact *C. parvum* oocysts:

In trial 1, nitazoxanide at concentrations of 10, 1 and 0.1 resulted in parasite inhibition levels of 94.4, 77.2 and 51.8%, respectively, and cell toxicity levels of 65.1, 8.3 and 19.3% respectively. Although nearly complete inhibition of parasite infection occurred in 10 μg/ml, a high toxicity rating was evident. At 1 μg/ml of nitazoxanide, parasite inhibition and cellular toxicity compared favorably to paromomycin at a concentration of 2 mg/ml (77.2% parasite inhibition and 8.3% toxicity for nitazoxanide at 1 μg/ml compared to 51% parasite inhibition and 23.8% cell toxicity for paromomycin at 2 mg/ml).

In trial 2, the drug was modified to obtain better dose distribution with minimum toxicity. Consequently, the cultures remained viable for 48 hours instead of 24 hours as in trial 1. Incubation for 48 hours clearly resulted in higher relative cell toxicity as evident form examination of paromomycin in both trials. The 20 μg/ml concentration of nitazoxanide was still too toxic at 48 hours incubation although the cell monolayer appeared still intact. It is possible that high toxicity which must affect cell function also impacts parasite infection/development. At 2 μg/ml of nitazoxanide, there was a considerable inhibition of the parasite infection with relatively low cellular toxicity. Further dilutions also resulted in significant inhibition and low toxicity. At a drug concentration of 2 μg/ml, moderate cell toxicity and inhibitory activity of 94.90% indicates that nitazoxanide at 2 μg/ml is superior to paromomycin for *in vitro C. parvum* infection at 2 mg/ml (e.g. 1000 times higher concentration).

**EXAMPLE III**

*CRYPTOSPORIDIUM PARVUM*

*In vitro* Dose and Storage Information:

Stocks of nitazoxanide and desacetyl-nitazoxanide (NTZ and NTZdes) were tested against intact *C. parvum* oocysts and excysted sporozoite infected cell monolayers at concentrations 10, 1, 0.1 and 0.01 μg/ml. Each compound was dissolved in 100% dimethyl sulfoxide (DMSO) and diluted to the desired concentrations with sterile DMEM. Each concentration of nitazoxanide and the media controls contained 0.025% DMSO as a constant.

The experiment used a cell culture of MDBKF5D2 Cells grown in 7 mm chambers, and as *Cryptosporidium parvum:* GCH1 oocysts, 5 X $10^4$ per well, and was conducted to compare paromomycin (positive control) against nitazoxanide (experimental drug). Materials included Immune Anti-*Cryptosporidium parvum* Sporozoite Rabbit Serum (0.1%) and Fluorescein-Conjugated Goat Anti-Rabbit Antibody (1%).

Toxicity Testing Assay:

200μl of medium containing nitazoxanide solution at the pre-mentioned concentrations and the proper controls were introduced into two wells of a 96 well plate containing confluent MDBKFD2 cell monolayers and two wells without monolayers. The drug was incubated on the monolayers at 37°C and 8% $CO_2$. At 48 hours, MTS (Owen's solution) and PMS were added to each well at concentrations of 333 μg/ml and 25 μM respectively. The plate was returned to the incubator in the dark to develop for 2 hours. At 2 hours, 100 μl of each supernatant was transferred to a new microtiter plate and read in the ELISA reader at 490 nm. Results were recorded and analyzed. Percent toxicity was calculated by subtracting the mean optical density (OD) of the drug supernatants from the mean OD of the medium control supernatants (no drug), dividing by the OD of the medium control and multiplying by 100.

$$\frac{\text{OD medium - OD drug x 100}}{\text{OD medium}}$$

Cytotoxicity scores were assigned as follows: 0.5% toxicity = 0, 6-25% toxicity = 1, 26-50% toxicity = 2, 51-75% toxicity = 3 and 76-100% toxicity =- 4. As a standard, cytotoxicity scores of 0 or 1 are to be considered acceptable levels of toxicity. Toxicity scores of 2, 3 or 4 are considered a high level or toxicity to the cell monolayer.

Intact C. *parvum* oocyst Assay:

5 x 104 C. *parvum* oocysts per well are incubated in the pre-mentioned concentrations of nitazoxanide at 37°C (8% $CO_2$) on confluent MDBKF5D2 cell monolayers. The level of infection in each well was determined and computer analyzed by an immunofluorescence assay at 48 hours. Percent Inhibition was calculated by subtracting the mean parasite count/field in the drug test wells from the mean parasite count/field in medium control (no drug), dividing by the medium control count and multiplying by 100.

$$\frac{\text{Medium control count - Drug test count}}{\text{Medium control count}} \text{ x 100}$$

Results:

*C. parvum* Oocysts Assay (48 hr.)

| Drugs | Conc | Parasite | ±SD | Tox/ OD | ±SD | %Inhib | %Tox | Score |
|---|---|---|---|---|---|---|---|---|
| **Aqueous Media** | 0 | 681.58 | ±271.02 | 2.024 | ±0.18 | 0 | 0 | 0 |
| **Paromomycin** | 2000 | 115.75 | ±44.65 | 1.219 | ±.009 | 83.02 | 39.79 | 2 |

(continued)

| Drugs | Conc | Parasite | ±SD | Tox/ OD | ±SD | %Inhib | %Tox | Score |
|---|---|---|---|---|---|---|---|---|
| **0.025% DMSO Media** | 0 | 628.50 | ±171.94 | 1.799 | ±1.45 | 0 | 0 | 0 |
| **NTZ** | 10 | 11.75 | ±7.33 | .413 | ±0.13 | 98.13 | 77.07 | 4 |
| | 1 | 39.67 | ±13.13 | 1.618 | ±.326 | 93.69 | 10.09 | 1 |
| | 0.1 | 643.42 | ±229.73 | 1.878 | ±.154 | ≤0 | ≤0 | 0 |
| | 0.01 | 714.33 | ±194.79 | 1.617 | ±.072 | ≤0 | 10.12 | 1 |
| **New NTZdes** | 10 | 13.75 | ±6.66 | .337 | ±.005 | 97.81 | 81.27 | 4 |
| | 1 | 39.92 | ±13.49 | 1.710 | ±.033 | 93.65 | 4.97 | 0 |
| | 0.1 | 649.86 | ±152.19 | 1.506 | ±.119 | ≤0 | 16.29 | 1 |
| | 0.01 | 749.33 | ±139.49 | 1.721 | ±.144 | ≤0 | 4.36 | 0 |

Conc. - µg/ml; Parasite - Mean parasite count/field (12 fields analyzed); %Inhib - Percent inhibitation of parasite infection; % Tox- Percent toxicity to cells by the drug.

It can be seen from the above that the Inhibitory activity of NTZdes was the same as NTZ of Example II. Both nitazoxanide and desacetyl nitazoxanide were equally effective in vitro against *Cryptosporidium parvum* when tested in parallel with 98 and 94% inhibitions obtained with 10 and 1 µg/ml for each compound respectively. For nitazoxanide 1 µg/ml was the lowest concentration giving more than 90% of inhibition while 50% inhibition could be obtained with lower concentrations of nitazoxanide such as 0.2, 0.1 and 0.02 µg/ml. In the same experimental condition paromomycin used as positive control was 2,000 times less effective with inhibitory concentrations ranging form 51 to 83% at a concentration of 2,000 µg/ml.

## EXAMPLE IV

*E. INTESTINALIS AND V. CORNEA*

2RK-13 cells (rabbit kidney cell line) were added to 24-well culture plates at a concentration of 2.6 x 105 cells per well (1.0 ml medium; RPMI 1640 with 2 mM L-glutamine and 5% heat-inactivated fetal bovine serum). Dishes were incubated at 37°C in a $CO_2$ incubator overnight at which time the wells were confluent (with one doubling, would estimate 5 x $10^5$ cells per well).

*Septata intestinalis* (tissue culture-derived) organisms were added to the host cells at a 3:1 ratio compared with the estimated host cells or at 15. x $10^6$ organisms per well. This ratio resulted in approximately 50% of the host cells becoming infected).

Drugs were dissolved in DMSO, water or methanol (depending on solubility) to generate stocks of 1.0 mg/ml. Stocks were stored at -70°C. Dilutions used in experiments are made in complete tissue culture medium. All dilutions are tested in triplicate well.

Medium is replaced every three-to-four days (containing freshly-diluted drugs).

On day six (after adding parasites and drugs), the cells are examined for toxicity. Control cells given drugs but no parasites are examined for confluency, morphology of cells, and presence of dead or floating cells. Cells incubated with parasites only are examined to confirm that parasites are infectious (i.e. presence of parasitophorous vacuoles). Cells incubates the parasites and drugs are evaluated for host cell toxicity and relative numbers of parasitophorous vacuoles (i.e. high, medium, or low).

On day ten, 100 µl of 10% SDS (0.5% final concentration) was added to the culture wells to disrupt host cell membranes and cause release of the microsporidia. The total number of parasites present in each well was determined by counting an aliquot on a hemacytometer. Results are expressed as percent inhibition (relative to infected cells given no drug).

The results are shown in Figs. 1-4.

## EXAMPLE V

*TOXOPLASMA GONDII*

Nitazoxanide and desacetyl nitazoxanide were tested against parasites, and more specifically, RH strain of *Toxoplasma gondii*, maintained by serial passages in mice. Cell cultures of MRC5 fibroblasts (Bio-Merieux, France) cultured in 96-well microplates were inocultated with *T. gondii.* 200 fresly harvested tachyzoites were added into each culture well, except in 8 control wells (negative controls). After 4 hours of incubation, drug dilutions were added into the cultures.

Nitazoxanide (NTZ) and desacetyl nitazoxanide (dNTZ) were tested at concentrations ranging between $8.10^{-4}$ and 40 mg/L. Drugs were initially dissolved in DMSO, at a concentration of 2 mg/mL, then serial dilutions were prepared in the culture medium. No precipitate was observed.

Drug dilutions were added into the cultures (8 wells for each dilution) then culture plates were incubated for 72 hours. Cultures were then fixed with cold methanol. Assessment of growth of *T. gondii* was performed buy ELISA using a peroxydase labeled rabbit anti *T. gondii* antibody. Optical density values were recorded for each well.

Resuts are presented by plotting the OD values obtained for each culture well, vs the concentration of the drug in the culture. Statistical analysis consisted in regression analysis with 95% confidence interval and determination of dose-response curves, from the OD values generated for each drug.

One plate was stained with Giemsa to examiner the cytopathic effect in the cultures.

Three separate experiments were realized. In each experiment, two culture plates were used for each compound; in each culture plate, 8 replicate wells were used for each drug concentration.

Results:

Similar results were obtained in the three sets of experiments. Graphic representations of the results of one representative experiment for each drug are shown on Figs. 5 a,b,c and 6 a,b,c.

Nitazoxanide (Figs. 5 a,b,c):

No inhibitory effect was noted for concentrations ranging between $10^{-4}$ mg/l and 0.3 mg/L. A significant effect was noted for concentration ≥0.6 mg/L, with a complete inhibition of *Toxoplasma* growth for concentrations ≥2.5 mg/L. However, a marked toxicity was noted on the cell monolayer for concentrations ≥2.5 mg/L.

Microscopic examination of the monolayer showed that NTZ, at a concentration of 1.25 mg/L induced cytopathic effect on the parasitized cells, with enlargement of the parasitophorous vacuole and reduction of the number of the intra-celular parasites. From regression analysis, the 50% inhibitory concentration could be estimated at 1.2 mg/L.

Deacetyl nitazoxanide (Figs. 7 a,b,c):

Similar results were obtained with deacetyl nitazoxanide: no effect for concentrations ranging between $10^{-4}$ mg/L and 0,3 mg/L, inhibition for concentration ≥0.6 mg/L, and marked toxicity for concentration ≥ 2.5 mg/L. The 50% inhibitory concentration could be estimated at 1.2 mg/L.

The results obtained were reproducible over three separate experiments, with assessment of the drug inhibitory effect on repeated cultures for each drug concentration.

For both NTZ and deacetyl NTZ, a marked inhibition of *Toxoplasma* growth could be observed at concentrations of approximately 1.2 mg/L, with alteration of the parasitophorous vacuole but no marked alteration of the parasite itself.

These results indicate that these drugs have good activity against *T. gondii,* and that a therapeutic effect can be expected in vivo based on obtaining a concentration of approximately 1 mg/L in serum or tissues.

## EXAMPLE VI

*MYCOBACTERIA*

Nitazoxanide was found to have antimicrobial activity against TB organisms. The following table shows an assay for MIC of nitazoxanide and tizoxanide against *Mycobacterium intracellular* by agar dilution technique. These results are based upon several experiments, each of which took about 3 weeks for the agar dilution method with Middlebrook agar. The data obtained indicate that nitazoxanide has an MIC against the Mycobacteria of 2 μg/ml and tizoxanide has an MIC of 4μg/ml, using a standard strain of *Mycobacterium intracellular* from ATCC, using the standard

agar dilution assay.

| MICs of Nitazoxanide and tizoxanide to *Mycobacteia intracellulare* | |
|---|---|
| MIC | |
| Nitazoxanide | 2 μg/ml |
| Tizoxanide | 4 μg/ml |
| *MICs were determined by standard agar dilution using Middlebrook 7H11 agar for 3 weeks. *M. intracellular* ATCC 13950, a standard strain, was used for this experiment. | |

Fig. 7 is a chart based upon the assay for nitazoxanide effectiveness against mycobacteria growing in a liquid broth. We used the MTS colorimetric assay which permits us to determine growth in 4 hours rather than 3 weeks as with the agar counting method. As can be seen from the data in Fig. 7, when nitazoxanide was added at the 72 hr after culture was initiated, there was an immediate effect on continued growth as compared to the growth in control medium alone. The 3 μg/ml dose of nitazoxanide stops growth for the next 24 hrs and then there is a slow growth afterwards for the next 2 days. The 50 μg/ml dose was completely bacteriostatic throughout the 144 hours of the culture.

**EXAMPLE VII**

*CRYPTOSPORIDIUM PARVUM*

The effect of nitazoxanide was tested against *Cryptosporidium parvum* in experimentally infected mice. Nitazoxanide was supplied by Romark Laboratories, L.C. in Tampa, Florida.

The total human dose (1 g/day for 7 days i.e. 7 g) was modified for use for mice according to Paget and Barnes. The human dose was multiplied by 0.0026 for mice (weighing approximately 20 grams) to obtain he total amount of the drug needed for each host morning and evening for 7 consecutive days. Each mouse received 2.6 mg/day (7000 mg x 0.0026/7). The doses were administered by mouth using a plastic syringe equipped with around tip needle.

Twenty (20) 2-day old suckling mice were infected by oral administration of 100,000 oocysts of *Crytospordium parvum* obtained from infected calves. Before being administered to mice, the oocysts were concentrated using a sugar solution according to the technique described by Fayer & Ellis. Rectal swabs from each mouse were obtained and examined daily using the modified Niehl-Neelsen staining technique described by Graczyk et al. Oocysts shedding appeared in feces 2 days after the oral infection of the animals. On the third day following infection of the animals, 10 mice received 1.3 mg of nitazoxanide, morning and evening, for 7 consecutive days while the 10 remaining mice were kept as untreated controls. Rectal swabs were obtained daily for each of the 7 days of treatment and for each of the 7 days following the end of treatment. The oocysts were suspended in oil and counted per 100 fields under a microscope.

Results:

The results shown in the following Table clearly indicate that nitazoxanide administered at a daily dose of 2.6 mg/day for 7 consecutive days was effective against *Cryptosporidium parvum* in reducing the number of oocysts in the feces of the infected mice when compared to the control animals. The test drug decreased the oocysts shedding in 6 of the 10 treated mice at the end of the third day of treatment. At the end of treatment of Day 7, there was a complete reduction of the oocyst shedding, all treatment animals having negative fecal examination when compared to untreated control mice. This effect lasted at least for 7 days after treatment as shown by negative examinations observed on days 3 and 7 after the end of treatment.

| | NO. OF OOCYST DETECTED PER OIL IMMERSION FIELD | | | | | | | |
|---|---|---|---|---|---|---|---|---|
| | At 3rd day of treatment | | At last day of treatment | | At 3rd day post-treatment | | At 7th day post-treatment | |
| Mice No. | Control group | Treated group | Control group | Treated group | Control group | Treated group | Control group | Treated group |
| 1 | 3.0 | 0.0 | 5.0 | 0.0 | 4.0 | 0.0 | 2.0 | 0.0 |
| 2 | 4.0 | 0.0 | 4.0 | 0.0 | 3.0 | 0.0 | 1.0 | 0.0 |
| 3 | 6.0 | 0.0 | 5.0 | 0.0 | 4.0 | 0.0 | 0.5 | 0.0 |
| 4 | 3.0 | 2.0 | 3.0 | 0.0 | 2.0 | 0.0 | 1.0 | 0.0 |
| 5 | 5.0 | 2.0 | 3.0 | 0.0 | 3.0 | 0.0 | 0.5 | 0.0 |
| 6 | 3.0 | 0.0 | 4.0 | 0.0 | 5.0 | 0.0 | 2.0 | 0.0 |
| 7 | 3.0 | 0.0 | 5.0 | 0.0 | 4.0 | 0.0 | 1.0 | 0.0 |
| 8 | 5.0 | 1.0 | 5.0 | 0.0 | 1.0 | 0.0 | 0.5 | 0.0 |
| 9 | 3.0 | 3.0 | 3.0 | 0.0 | 2.0 | 0.0 | 1.0 | 0.0 |
| 10 | | 0.0 | 5.0 | 0.0 | 2.0 | 0.0 | 0.5 | 0.0 |
| Total | 35 | 8.0 | 4.2 | 0.0 | 30 | 0.0 | 10 | 0.0 |
| Mean | 3.5 | 0.8 | 4.2 | 0.0 | 3.0 | 0.0 | 1.0 | 0.0 |
| Efficacy | | 60% | | 100% | | 100% | | 100% |

## EXAMPLE VIII

*MYCOBACTERIUM*

Nitazoxanide was compared against izoniazide antibiotic. The protocol used BCG (Bacille de Calmette et Guerin) as a mycobacterium strain. The sensitivity of this strain was the same as that of *M. tuberculosis,* but this strain is more harmeless and thus did not require high level of containment of a tuberculosis agent.

4 mg/mouse per day in 0.2 ml of sunflower oil was administered to mice. The results in mice treated with nitazoxanide were comparable to the group receiving izoniazide.

| | $10^7$ | | | $10^5$ | | |
|---|---|---|---|---|---|---|
| | Spleen | Liver | Lungs | Spleen | Liver | Lungs |
| Nitazo | 1 575 000<br>800 000<br>875 000<br>950 000 | 1 575 000<br>1 550 000<br>1 550 000<br>750 000 | 57 500<br>122 500<br>30 000<br>75 000 | 68 250<br>65 000<br>75 000<br>60 000 | 70 000<br>87 500<br>35 000<br>60 000 | 50<br>75<br>150<br>50 |
| INH | 475 000<br>255 000<br>200 000 | 1 050 000<br>750 000<br>975 000 | 11 000<br>5 750<br>4 000 | 20 000<br>15 250<br>60 000<br>20 000 | 21 250<br>27 500<br>52 500<br>37 500 | 50<br>125<br>50<br>50 |
| PBS | 1 500 000<br>1 525 000<br>1 925 000<br>1 675 000 | 2 125 000<br>1 800 000<br>1 750 000<br>1 800 000 | 92 500<br>98 000<br>177 500<br>117 500 | 102 500<br>140 000<br>98 000<br>105 000 | 195 000<br>175 000<br>150 000<br>150 000 | 750<br>800<br>500<br>750 |

## EXAMPLE IX

*FASCIOLA HEPATICA*

The *in vitro* efficacy of nitazoxanide and desacetyl-nitazoxanide was tested against *Fasciola hepatica.*

Mature *F. hepatica* were recovered from the bile ducts of 3 calf livers condemned due to fasciolosis at the Louisiana Veterinary Medical Diagnostic Laboratory at the Hardy's Meat Packers, Bunkie, LA. Flukes were washed in sterile saline for 1 hour and transferred to sterile saline or RPMI (pH 7.4) for an additional 3 hours. Flukes were then held in sterile RPMI-rabbit serum (50:50 v/v) or sterile RPMI (pH 7.4) overnight at 37°C with 5% $CO_2$.

*In vitro* culture (37°C, 5% $CO_2$) was done according to a modification of the method of Ibarra and Jenkins (Z. Parasitenkd. 70:655-661, 1984). Using sterile technique, flukes were washed twice for 2-3 minutes in Hank's balanced salt solution (pH 7.2) and placed individually into wells of six-well Linbro culture plates containing 10 ml aliquots of the designated dilutions of the drug in culture media. The latter consisted of sterile 50:50 v/v RPMI-Rabbit serum with 2% Rabbit Blood plus 100 ppm Penicillin and 100 ppm Streptomycin. Only flukes that had normal activity and morphology were used.

Stock solutions of NTZ or its metabolite D-NTZ provided by Romark were dissolved in DMSO (2000 μg/ml) and diluted in culture medium, using 100 ml volumetric flasks, to produce the specified drug concentrations (100, 50, 25, 10, 5, 3, 1 μg/ml). Two control flukes were included in each replicate, one in unmediated culture media with RBC and one in unmedicated culture media with RBC.

Flukes were examined for the effects of drug treatment as evidenced by death, motility disturbances or morpholgic changes as compared to untreated control flukes, using a backlighted panel and a lighted 3X magnifying lens.

Results:

**Experiment 1:** For D-NTZ, flukes in the 50 and 100 μg treatments were moribund or dead within one hour. Four of 7 flukes in the 25 μg treatment were moribund, two were active, and one was sluggish within the first hour; all were dead except two sluggish flukes by three hours and only one sluggish fluke remained alive after four hours. AT 10 μg, reduced activity was noted at 1, 3 and 4 hours and all were moribund or dead by 7 hours. Reduced activity in some individuals was seen by 24 hours in the 5 μg and 3 μg groups with a somewhat slower onset at 3 μg; all were dead in the 3 and 5 μg treatment wells by 50 hours except one sluggish fluke in each group. Some slowing of activity was noted in the 1 μg group at 42-74 hours and only 3 active and one moribund fluke remained alive at 91 hours; at 115 hours only one sluggish fluke remained in the 1 /g group. Mortality in the control with RBC group was observed at 66 hours (one fluke), 91 hours (one fluke) and 115 hours (four flukes). In the Control without RBC group, all were alive at hour 91 and one was dead at hour 115.

**Experiment 2:** For NTZ, somewhat greater activity was noted by earlier effects on motility score and mortality in the 8 replicates as compared to results for D-NTZ. In the 100, 50 and 25 μg groups all flukes were dead or moribund except one fluke at 1 hour in the 25 μg group it was dead at 3 hour. Dose-related reduction in motility was seen in each of the other medicated groups beginning at hour 1. At 10 μg, only one fluke survived to 16 hours. In the 5 μg group, only 3 flukes were active at hour 6 and none were active at 16 hours. By 23 hours, only 2 sluggish flukes in the 3 μg group remained alive; these were dead by hour 41. For the 1 μg group, one fluke died by hour 16, three by hour 41 and five by hour 74; 3 flukes remained active at hour 91 and one fluke had activity at hour 115. In the Control with RBC group, 7 of 8 flukes were alive at hour 74, 3 were alive at hour 91 and 2 survived at hour 115. In the Control without RBC group, 6 of 8 flukes had activity at hour 74, 4 were active at hour 91 and two remained active at hour 115.

Fluke death in the high dose groups (25, 50, 100 μg) was rapid and associated with contraction and ventral 'curling'. At lower medication levels, most flukes slowed for a period and were more relaxed and 'flattened' when moribund or dead.

Contamination became limiting on experimental results in some replicates beginning at hour 91. For the D-NTZ experiment, major bacterial or fungal overgrowth and associated mortality in two replicate plates occurred by hour 115. For the NTZ experiment, overgrowth and fluke mortality in entire replicate plates occurred by hour 91 (two replicates), and hour 115 (5 replicates). Hour 139 observations were not considered valid because of general contamination of most plates.

Conclusions:

Strong flukecidal efficacy by nitazoxanide is suggested by experiments with both drugs tested. Somewhat greater flukecidal activity against *F. hepatica* was observed for nitaxoxanide than for desacetyl-nitazoxanide, the main metabolite, which is thought to be active at the hepatic level.

Rapid fluke death occurs within 1 hour at *in vitro* D-NTZ medication rates of >50 μg, within 4 hours at 25 μg

and by 6-7 hours at 10µg. Ten µg, may be an appropriate single treatment target drug delivery rate if pharmacokinetic data indicate tissue levels are maintained for >6-8 hours after a single treatment.

Strong flukecidal activity by 74 hours (three days) was observed for both compounds at the 3 and 5 µg dose rates. Prolonged survival approaching, but not equal to unmedicated control flukes was observed at the 1µg dose level; delivery of this level of drug to flukes in hepatic tissues for 3 to 4 days may therefore have inadequate therapeutic effect on parasites.

**EXAMPLE X**

*FASCIOLA GIGANTICA*

Nitazoxanide was tested against immature and mature *Fasciola gigantica* in experimentally infected rabbits.

*Fasciola gigantica* encysted metacercariae (EMC) were collected on cellophane sheet after 28-35 days from infection of *L. calludi* snails by *Fasciola gigantica* miracidium using the technique described by Abdel-Ghany where snails were exposed daily to artificial light, for 30 minutes, in clean dechlorinated tap water. The resulting encysted metacercaraie (EMC) were preserved at 4°C in a refrigerator for 5 to 8 days under the surface of water until they were used to infect experimental animals.

Forty (40) Boscat rabbits, weighting 1.5 to 2 kg each, were included in the study and allocated to two treatment groups of 20 animals.

Animals from Group 1 were orally infected with 35-40 encysted metacercarae wrapped in a leaf of lettuce and pushed on the root of the tongue of the animals. The mouths of the animals were held closed by hand until the encysted metacercariae were swallowed. These Group 1 animals were used to test the efficacy of nitazoxanide against immature stages (4-5 weeks old) of *Fasciola gagantica*.

Animals from Group 2 were orally infected as indicated above with 10-15 encysted metacercariae and were used to test the efficacy of nitazoxanide against the early mature flukes (>10 weeks old).

Ten animals from Group 1 received 35 mg of nitazoxanide, morning and evening, for 7 consecutive days 4 weeks after their infection at the immature stage of the parasite cycle. The ten remaining animals in Group 1 were kept as untreated controls.

Ten animals from Group 2 received 35 mg of nitazoxanide, morning and evening, for 7 consecutive days 10 weeks after their infection at the mature stage of the parasite. The 10 remaining animals in Group 2 were kept as untreated controls.

All animals were fed with dry ration until the end of the experiment.

Seven days after administration of the last dose of nitazoxanide, all rabbits from each group were sacrificed. The surface of the liver was examined for the presence of necrotic migrating furrows especially at the immature stage of the parasite cycle. These necrotic areas were examined using two surgical needles in order to extract the juvenile migrating flukes according to the technique described by El-Bahy. The livers were sliced in small pieces especially around the migrating furrows and macerated under a microscope in order to extract the existing flukes. The abdominal cavity and the visceral surfaces were washed with warm water. The water was then collected, sieved and examined for identification of juvenile flukes. All the collected parasites as well as parts of them were counted in both treated and untreated animal in both Groups 1 and 2. Living flukes appeared pin in color, translucid showing intact teguments, easily extractable from the tissue of the livers using warm water, while dead flukes were grayish in color loose and showed a broken necrotic surface. The efficacy of nitazoxanide was calculated by using the formula indicated below:

$$\text{\% efficacy } \frac{a - b}{a} \times 100$$

Where:

a = the number of flukes recovered from feces in the control animals
b = the number of flukes recovered from feces in the treated animals.

**Results**

The results of the study as indicated in Table 7 show a marked decrease in the number of immature flukes recovered from the liver of rabbits in the treated group when compared in the control group. The means percentage of reduction was calculated as 46.77% (range: 40-60%).

Table 7:

| Efficacy of nitazoxanide against immature (4-week/old) F. gigantica in experimentally infected rabbits | | | |
|---|---|---|---|
| **No. of flukes extracted from liver of** | | | |
| **Rabbit No.** | **Untreated Control** | **Treated Rabbits** | **Efficacy %** |
| 1 | 7 | 4 | 42% |
| 2 | 7 | 4 | 42% |
| 3 | 6 | 3 | 50% |
| 4 | 8 | 4 | 50% |
| 5 | 5 | 3 | 40% |
| 6 | 5 | 2 | 60% |
| 7 | 5 | 3 | 40% |
| 8 | 6 | 3 | 50% |
| 9 | 8 | 4 | 50% |
| 10 | 5 | 3 | 40% |
| Mean | 6.2 | 3.3 | 46.77% |

At the early mature stage of their infection, nitazoxanide showed a complete effect (100% reduction) and no worms could be seen after examination of the liver of the treated rabbits in comparison with the untreated control animals as shown in Table 8.

Table 8:

| Efficacy of nitazoxanide against early mature (10-week/old) F. gigantica in experimentally infected rabbits | | | |
|---|---|---|---|
| **No. of flukes extracted from liver of** | | | |
| **Rabbit No.** | **Untreated Control** | **Treated Rabbits** | **Efficacy %** |
| 1 | 4 | 0.0 | 100% |
| 2 | 4 | 0.0 | 100% |
| 3 | 3 | 0.0 | 100% |
| 4 | 3 | 0.0 | 100% |
| 5 | 2 | 0.0 | 100% |
| 6 | 2 | 0.0 | 100% |
| 7 | 2 | 0.0 | 100% |
| 8 | 3 | 0.0 | 100% |
| 9 | 3 | 0.0 | 100% |
| 10 | 3 | 0.0 | 100% |
| Mean | 2.9 | 0.0 | 100% |

Nitazoxanide administered as a 70 mg/day dose for 7 consecutive days is moderately effective against immature stage of *Fasciola gigantica* and completely effective against the early mature stage of the parasite.

**EXAMPLE XIII**

*SCHISTOSOMA*

Nitazoxanide was tested against *Schistosoma mansoni* and *Schistosoma hematobium* in experimentally infected mice.

Forty (40) white mice, weighing 30 to 50 grams were allocated to two treatment groups of 20 animal per group. The first group was infected with 300-500 *Schistosoma mansoni* free active cercariae suspended in 0.25 ml of distilled water and administered to each mouse by intraperitoneal injection. The second group was infected in the same manner but with *Schistosoma hemotobium* cercariae. These two groups were then kept for a total of 70 days in the laboratory.

Seventy days after infection of the animals, ten mice from each group were treated with nitazoxanide as a 1.3 mg oral dose administered, morning and evening, for 7 consecutive days. Seven days after the end of treatment tall mice were sacrificed and the worms were extracted from the liver of each animal by perfusion using tepid water (37°C). The extracted schistosomes were counted for all treatment and control animals. The efficacy of nitazoxanide was calculated busing the formula indicated below:

$$\% \text{ efficacy } \frac{a - b}{a} \times 100$$

Where:

a = the number of schistosomes recovered from feces in the control animals
b = the number of schistosomes recovered from feces in the treated animals

## Results

The results shown in Tables 9 and 10 clearly indicate that nitazoxanide administered at a daily dose of 2.6 mg/day for 7 consecutive days was more effective against *Schistosoma hematobium* where a worm reduction of 82.85% was observed when compared to the control animals while against *Schistosoma mansoni,* the worm reduction only achieved 59.91% versus the control mice. These results are consistent with those report by Abaza et al. in patients where nitazoxanide was not effective against *S. mansoni* as shown by nitazoxanide posttreatment positive egg-counts.

Table 9:

| Efficacy of nitazoxanide against mature (13-week/old) Schistosoma mansoni in mice | | |
|---|---|---|
| **No. of flukes removed from liver of** | | |
| **Mice No.** | **Untreated Control** | **Treated Mice** |
| 1 | 21 | 10 |
| 2 | 29 | 9 |
| 3 | 32 | 10 |
| 4 | 26 | 11 |
| 5 | 24 | 13 |
| 6 | 19 | 10 |
| 7 | 20 | 9 |
| 8 | 24 | 12 |
| 9 | 22 | 8 |
| 10 | 30 | 7 |
| Total | 247 | 99 |
| Mean/Mouse | 24.7 | 9.9 |
| Efficacy | | 59.91 |

Table 10:

| Efficacy of nitazoxanide against mature (13-week/old) Schistosoma hematobium in mice | | |
|---|---|---|
| **No. of flukes removed from liver of** | | |
| **Mice No.** | **Untreated Control** | **Treated Mice** |
| 1 | 18 | 3 |
| 2 | 16 | 3 |
| 3 | 14 | 2 |
| 4 | 19 | 2 |
| 5 | 12 | 4 |
| 6 | 10 | 4 |
| 7 | 13 | 2 |
| 8 | 12 | 2 |
| 9 | 17 | 0.0 |
| 10 | 9 | 2 |
| Total | 140 | 24 |
| Mean/Mouse | 14 | 2.4 |
| Efficacy | | 82.85 |

## Claims

1. A pharmaceutical composition containing as active agent, at least one compound selected from the group consisting of:
a compound of formula I

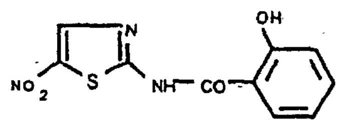

and a compound of formula II

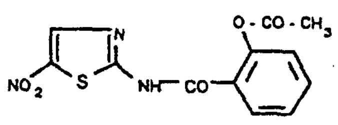

and further containing a stability improving amount of a pharmaceutically acceptable acid.

2. A pharmaceutical composition according to claim 1, wherein the pharmaceutically acceptable acid is selected from the group consisting of citric acid, glutamic acid, succinic acid, ethanesulfonic acid, acetic acid, tartaric acid, ascorbic acid, methanesulfonic acid, fumaric acid, adipic acid, malic acid and mixtures thereof.

3. The composition of claim 2, wherein said pharmaceutically acceptable acid is citric acid.

4. The composition of claim 2, wherein said pharmaceutically acceptable acid is ascorbic acid.

**5.** A pharmaceutical composition containing as active agent
a compound of formula II

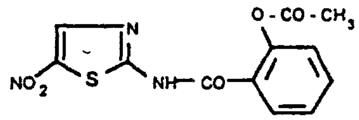

and a pharmaceutically acceptable acid in an amount sufficient to render the pH of said pharmaceutical composition between 2 and 6 when said pharmaceutical composition is contacted with water, wherein said active agent is in the form of solid particles, and wherein the ratio of the weight of the pharmaceutically acceptable acid to the weight of said solid particles is between 0.01 and 0.5.

**6.** The composition of claim 5, wherein the ratio of the weight of pharmaceutically acceptable acid to the weight of said active solid particles is between 0.03 and 0.2.

**7.** The composition of claim 2, wherein said composition is in a solid dosage form, and wherein said active particles are granulated in the presence of at least a granulating agent to form granulated active solid particles.

**8.** The composition of claim 5, wherein said active particles prior to granulating have a particle size smaller than 200 µm and a mean particle size greater than 10 µm.

**9.** The composition of claim 8, wherein said mean particle size of said active particles is between 10 and 100µm.

**10.** The composition of claim 7, wherein said granulating agent is selected from the group consisting of polyvinylpyrrolidone, water, alcohol, sucrose, hydroxyl cellulose and mixture thereof.

**11.** The composition of claim 7, wherein in said granulated active solid particles, the ratio by weight of pharmaceutically acceptable acid/weight of active agent is between 0.01 and 0.5.

**12.** The composition of claim 7, wherein said pharmaceutically acceptable acid is selected from the group consisting of citric acid, glutamic acid, succinic acid, ethanesulfonic acid, acetic acid, tartaric acid, ascorbic acid, methanesulfonic acid, fumaric acid, adipic acid, malic acid and mixtures thereof.

**13.** The composition of claim 2, wherein said composition is in the form of a suspension of solid particles of at least one compound of formula I and formula II in a liquid.

**14.** The composition of claim 13, wherein said active particles prior to forming said suspension are granulated in the presence of at least a granulating agent to form granulated active solid particles.

**15.** The composition of claim 13, wherein said liquid is water.

**16.** The composition of claim 13, wherein the pH of said suspension is between 2 and 6.

**17.** The composition of claim 13, wherein the pH of said suspension is between 3 and 5.

**18.** The composition of claim 2, wherein said composition is in the form of a paste comprising active particles of at least one compound of formula I and formula II, a wetting agent, and a thickener.

**19.** The composition of claim 18, wherein said pharmaceutically acceptable acid is selected from the group consisting of citric acid, glutamic acid, succinic acid, ethanesulfonic acid, acetic acid, tartaric acid, ascorbic acid, methanesulfonic acid, fumaric acid, adipic acid, malic acid, and mixtures thereof.

**20.** The composition of claim 18, wherein said active particles have a particle size smaller than 200 µm, less than 10 % by weight of said particles have a size larger than 100 µm, less than 50 % of said particles have size larger than 50 µm, and less than 10 % by weight of said particles having a size less than 5 µm.

**Patentansprüche**

1. Pharmazeutische Zusammensetzung, die als Wirkstoff wenigstens eine Verbindung enthält, die ausgewählt ist aus der Gruppe, bestehend aus:

   einer Verbindung von Formel I

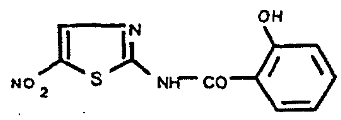

   und einer Verbindung von Formel II

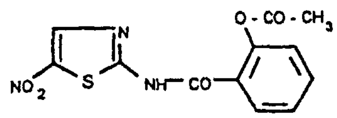

   und die weiter eine die Stabilität verbessernde Menge einer pharmazeutisch annehmbaren Säure enthält.

2. Pharmazeutische Zusammensetzung nach Anspruch 1, **dadurch gekennzeichnet, daß** die pharmazeutisch annehmbare Säure ausgewählt ist aus der Gruppe, die aus Zitronensäure, Glutaminsäure, Bernsteinsäure, Ethansulfonsäure, Essigsäure, Weinsäure, Ascorbinsäure, Methansulfonsäure, Fumarsäure, Adipinsäure, Äpfelsäure und Mischungen derselben besteht.

3. Zusammensetzung nach Anspruch 2, **dadurch gekennzeichnet, daß** besagte pharmazeutisch annehmbare Säure Zitronensäure ist.

4. Zusammensetzung nach Anspruch 2, **dadurch gekennzeichnet, daß** besagte pharmazeutisch annehmbare Säure Ascorbinsäure ist.

5. Pharmazeutische Zusammensetzung, die als Wirkstoff eine Verbindung von Formel II

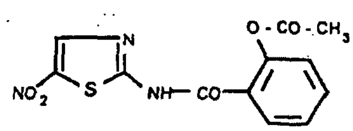

   und eine pharmazeutisch annehmbare Säure in einer Menge enthält, die ausreichend ist, um den pH besagter pharmazeutischen Zusammensetzung zwischen 2 und 6 einzustellen, wenn besagte pharmazeutische Zusammensetzung mit Wasser in Kontakt gebracht wird, wobei besagter Wirkstoff in der Form von festen Teilchen vorliegt und wobei das Verhältnis des Gewichts der pharmazeutisch annehmbaren Säure zum Gewicht besagter festen Teilchen zwischen 0,01 und 0,5 liegt.

6. Zusammensetzung nach Anspruch 5, **dadurch gekennzeichnet, daß** das Verhältnis des Gewichts der pharmazeutisch annehmbaren Säure zum Gewicht besagter aktiven festen Teilchen zwischen 0,03 und 0,2 liegt.

7. Zusammensetzung nach Anspruch 2, **dadurch gekennzeichnet, daß** besagte Zusammensetzung in einer festen Dosisform vorliegt und daß besagte aktive Teilchen in Gegenwart wenigstens eines Granulierungsmittels granuliert sind, um granulierte aktive feste Teilchen zu bilden.

8. Zusammensetzung nach Anspruch 5, **dadurch gekennzeichnet, daß** besagte aktive Teilchen vor dem Granulieren eine Teilchengröße von weniger als 200 µm und eine durchschnittliche Teilchengröße von mehr als 10 µm besitzen.

**9.** Zusammensetzung nach Anspruch 8, **dadurch gekennzeichnet, daß** besagte durchschnittliche Teilchengröße besagter aktiven Teilchen zwischen 10 und 100 µm liegt.

**10.** Zusammensetzung nach Anspruch 7, **dadurch gekennzeichnet, daß** besagtes Granulierungsmittel ausgewählt ist aus der Gruppe, die aus Polyvinylpyrrolidon, Wasser, Alkohol, Saccharose, Hydroxylcellulose und Mischungen derselben besteht.

**11.** Zusammensetzung nach Anspruch 7, **dadurch gekennzeichnet, daß** in besagten granulierten aktiven festen Teilchen das Verhältnis des Gewichts der pharmazeutisch annehmbaren Säure zum Gewicht des Wirkstoffs zwischen 0,01 und 0,5 liegt.

**12.** Zusammensetzung nach Anspruch 7, **dadurch gekennzeichnet, daß** besagte pharmazeutisch annehmbare Säure ausgewählt ist aus der Gruppe, die aus Zitronensäure, Glutaminsäure, Bernsteinsäure, Ethansulfonsäure, Essigsäure, Weinsäure, Ascorbinsäure, Methansulfonsäure, Fumarsäure, Adipinsäure, Äpfelsäure und Mischungen derselben besteht.

**13.** Zusammensetzung nach Anspruch 2, **dadurch gekennzeichnet, daß** besagte Zusammensetzung in der Form einer Suspension von festen Teilchen von wenigstens einer Verbindung von Formel I und Formel II in einer Flüssigkeit vorliegt.

**14.** Zusammensetzung nach Anspruch 13, **dadurch gekennzeichnet, daß** besagte aktive Teilchen vor dem Bilden besagter Suspension in Gegenwart wenigstens eines Granulierungsmittels granuliert sind, um granulierte aktive feste Teilchen zu bilden.

**15.** Zusammensetzung nach Anspruch 13, **dadurch gekennzeichnet, daß** besagte Flüssigkeit Wasser ist.

**16.** Zusammensetzung nach Anspruch 13, **dadurch gekennzeichnet, daß** der pH besagter Suspension zwischen 2 und 6 liegt.

**17.** Zusammensetzung nach Anspruch 13, **dadurch gekennzeichnet, daß** der pH besagter Suspension zwischen 3 und 5 liegt.

**18.** Zusammensetzung nach Anspruch 2, **dadurch gekennzeichnet, daß** besagte Zusammensetzung in der Form einer Paste vorliegt, die aktive Teilchen von wenigstens einer Verbindung von Formel I und Formel II, ein Benetzungsmittel und ein Verdickungsmittel umfaßt.

**19.** Zusammensetzung nach Anspruch 18, **dadurch gekennzeichnet, daß** besagte pharmazeutisch annehmbare Säure ausgewählt ist aus der Gruppe, die aus Zitronensäure, Glutaminsäure, Bernsteinsäure, Ethansulfonsäure, Essigsäure, Weinsäure, Ascorbinsäure, Methansulfonsäure, Fumarsäure, Adipinsäure, Äpfelsäure und Mischungen derselben.

**20.** Zusammensetzung nach Anspruch 18, **dadurch gekennzeichnet, daß** besagte aktive Teilchen eine Teilchengröße von weniger als 200 µm besitzen, weniger als 10 Gew.-% besagter Teilchen eine Größe von mehr als 100 µm besitzen, weniger als 50% besagter Teilchen eine Größe von mehr als 50 µm besitzen und weniger als 10 Gew.-% besagter Teilchen eine Größe von weniger als 5 µm besitzen.

## Revendications

**1.** Composition pharmaceutique contenant en tant que principe actif, au moins un composant choisi dans le groupe se composant de :

un composé de formule I

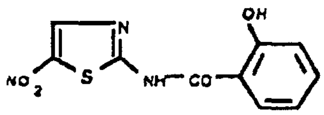

et un composé de formule II

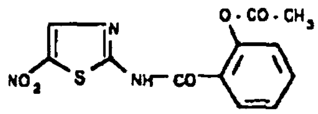

et contenant en outre une quantité améliorant la stabilité, d'un acide acceptable du point de vue pharmaceutique.

**2.** Composition pharmaceutique selon la revendication 1, dans laquelle l'acide acceptable du point de vue pharmaceutique est choisi dans le groupe se composant de l'acide citrique, l'acide glutamique, l'acide succinique, l'acide éthanesulfonique, l'acide acétique, l'acide tartrique, l'acide ascorbique, l'acide méthanesulfonique, l'acide fumarique, l'acide adipique, l'acide malique et les mélanges de ceux-ci.

**3.** Composition de la revendication 2, dans laquelle ledit acide acceptable du point de vue pharmaceutique est l'acide citrique.

**4.** Composition de la revendication 2, dans laquelle ledit acide acceptable du point de vue pharmaceutique est l'acide ascorbique.

**5.** Composition pharmaceutique contenant en tant que principe actif
un composé de formule II

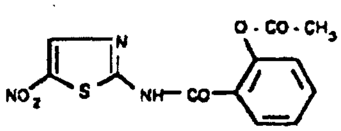

et un acide acceptable du point de vue pharmaceutique en une quantité suffisante pour faire en sorte que le pH de ladite composition pharmaceutique se situe entre 2 et 6 quand ladite composition pharmaceutique est au contact de l'eau, dans laquelle ledit principe actif se présente sous la forme de particules solides, et dans laquelle le ratio entre le poids de l'acide acceptable du point de vue pharmaceutique et le poids desdites particules solide se situe entre 0,01 et 0,5.

**6.** Composition de la revendication 5, dans laquelle le ratio entre le poids de l'acide acceptable du point de vue pharmaceutique et le poids desdites particules solides se situe entre 0,03 et 0,2.

**7.** Composition de la revendication 2, dans laquelle ladite composition se présente sous la forme d'un dosage solide, et dans laquelle lesdites particules actives sont granulées en présence d'au moins un agent de granulation pour constituer des particules solides actives granulées.

**8.** Composition de la revendication 5, dans laquelle lesdites particules actives ont, avant leur granulation, une taille de particules inférieure à 200 μm et une taille moyenne de particules supérieure à 10 μm.

**9.** Composition de la revendication 8, dans laquelle ladite taille moyenne de particules desdites particules actives se situe entre 10 et 100 μm.

10. Composition de la revendication 7, dans laquelle ledit agent de granulation est choisi dans le groupe se composant du polyvinylpyrrolidone, de l'eau, de l'alcool, du saccharose, de l'hydroxyl cellulose et des mélanges de ceux-ci.

11. Composition de la revendication 7, dans laquelle lesdites particules actives granulées, le ratio en poids de l'acide acceptable du point de vue pharmaceutique et en poids du principe actif se situe entre 0,01 et 0,5.

12. Composition de la revendication 7, dans laquelle ledit acide acceptable du point de vue pharmaceutique est choisi dans le groupe se composant de l'acide citrique, l'acide glutamique, l'acide succinique, l'acide éthanesulfonique, l'acide acétique, l'acide tartrique, l'acide ascorbique, l'acide méthanesulfonique, l'acide fumarique, l'acide adipique, l'acide malique et les mélanges de ceux-ci.

13. Composition de la revendication 2, dans laquelle ladite composition se présente sous la forme d'une suspension de particules solides d'au moins un composé de formule I et de formule II dans un liquide.

14. Composition de la revendication 13, dans laquelle lesdites particules actives avant de former ladite suspension sont granulées en présence d'au moins un agent de granulation pour former des particules solides actives granulées.

15. Composition de la revendication 13, dans laquelle ledit liquide est de l'eau.

16. Composition de la revendication 13, dans laquelle le pH de ladite suspension se situe entre 2 et 6.

17. Composition de la revendication 13, dans laquelle le pH de ladite suspension se situe entre 3 et 5.

18. Composition de la revendication 2, dans laquelle ladite composition se présente sous la forme d'une pâte comprenant des particules actives d'au moins un composé de formule I et de formule II, d'un agent mouillant et d'un épaississant.

19. Composition de la revendication 18, dans laquelle ledit acide acceptable du point de vue pharmaceutique est choisi dans le groupe se composant de l'acide citrique, l'acide glutamique, l'acide succinique, l'acide éthanesulfonique, l'acide acétique, l'acide tartrique, l'acide ascorbique, l'acide méthanesulfonique, l'acide fumarique, l'acide adipique, l'acide malique et les mélanges de ceux-ci.

20. Composition de la revendication 18, dans laquelle lesdites particules actives ont une taille de particules inférieure à 200 μm, moins de 10% en poids desdites particules ont une taille supérieure à 100 μm, moins de 50% desdites particules ont une taille supérieure à 50 μm, et moins de 10% en poids desdites particules ont une taille inférieure à 5 μm.

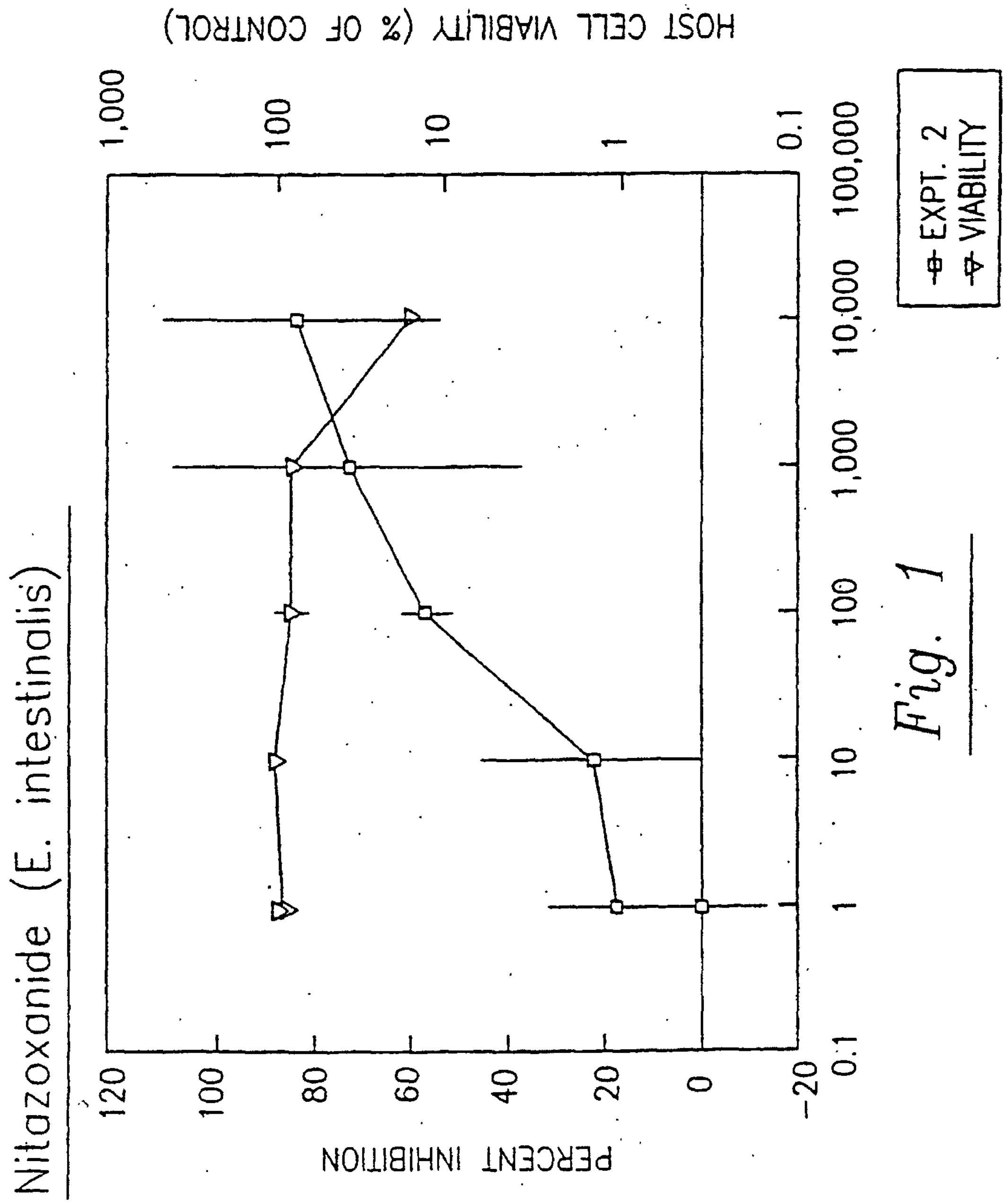
Nitazoxanide (E. intestinalis)
PERCENT INHIBITION
120
100
80
60
40
20
0
-20
0.1
1
10
100
1,000
10,000
100,000
HOST CELL VIABILITY (% OF CONTROL)
1,000
100
10
1
0.1
EXPT. 2
VIABILITY

*Fig. 1*

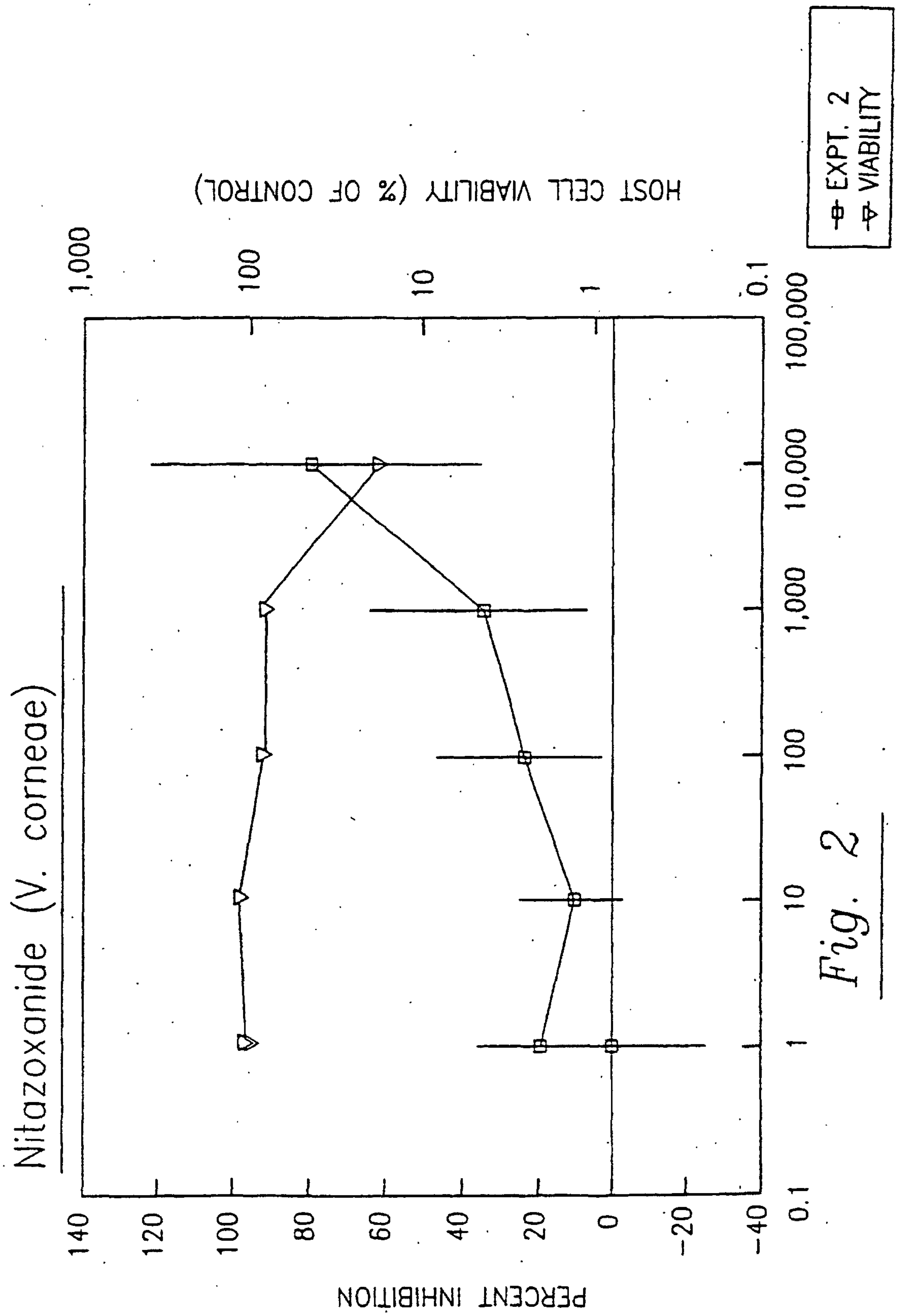
Nitazoxanide (V. corneae)
PERCENT INHIBITION
140
120
100
80
60
40
20
0
-20
-40
0.1
1
10
100
1,000
10,000
100,000
HOST CELL VIABILITY (% OF CONTROL)
1,000
100
10
1
0.1
EXPT. 2
VIABILITY

*Fig. 2*

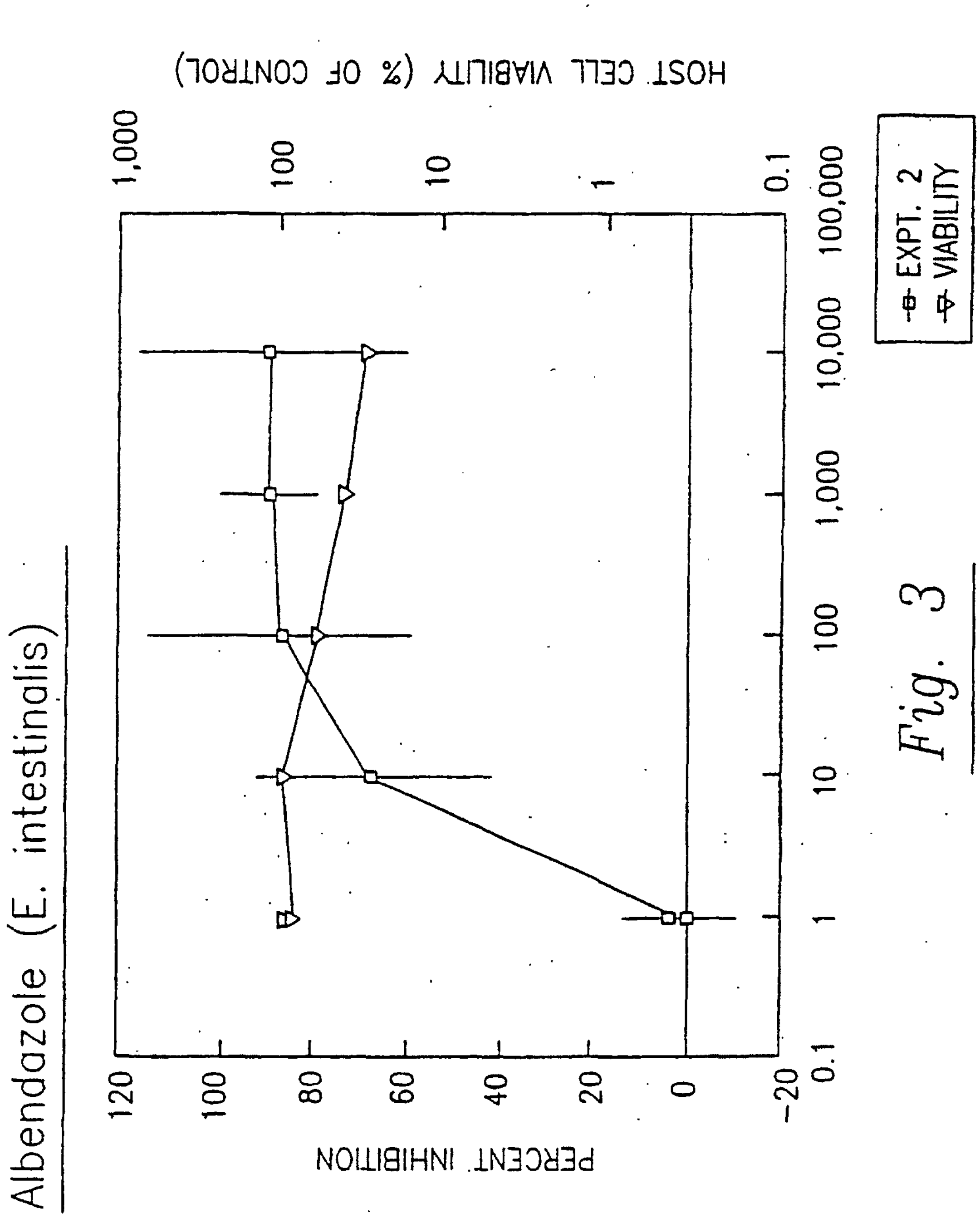
Albendazole (E. intestinalis)
PERCENT INHIBITION
HOST CELL VIABILITY (% OF CONTROL)
120
100
80
60
40
20
0
−20
0.1
1
10
100
1,000
10,000
100,000
1,000
100
10
1
0.1
EXPT. 2
VIABILITY

Fig. 3

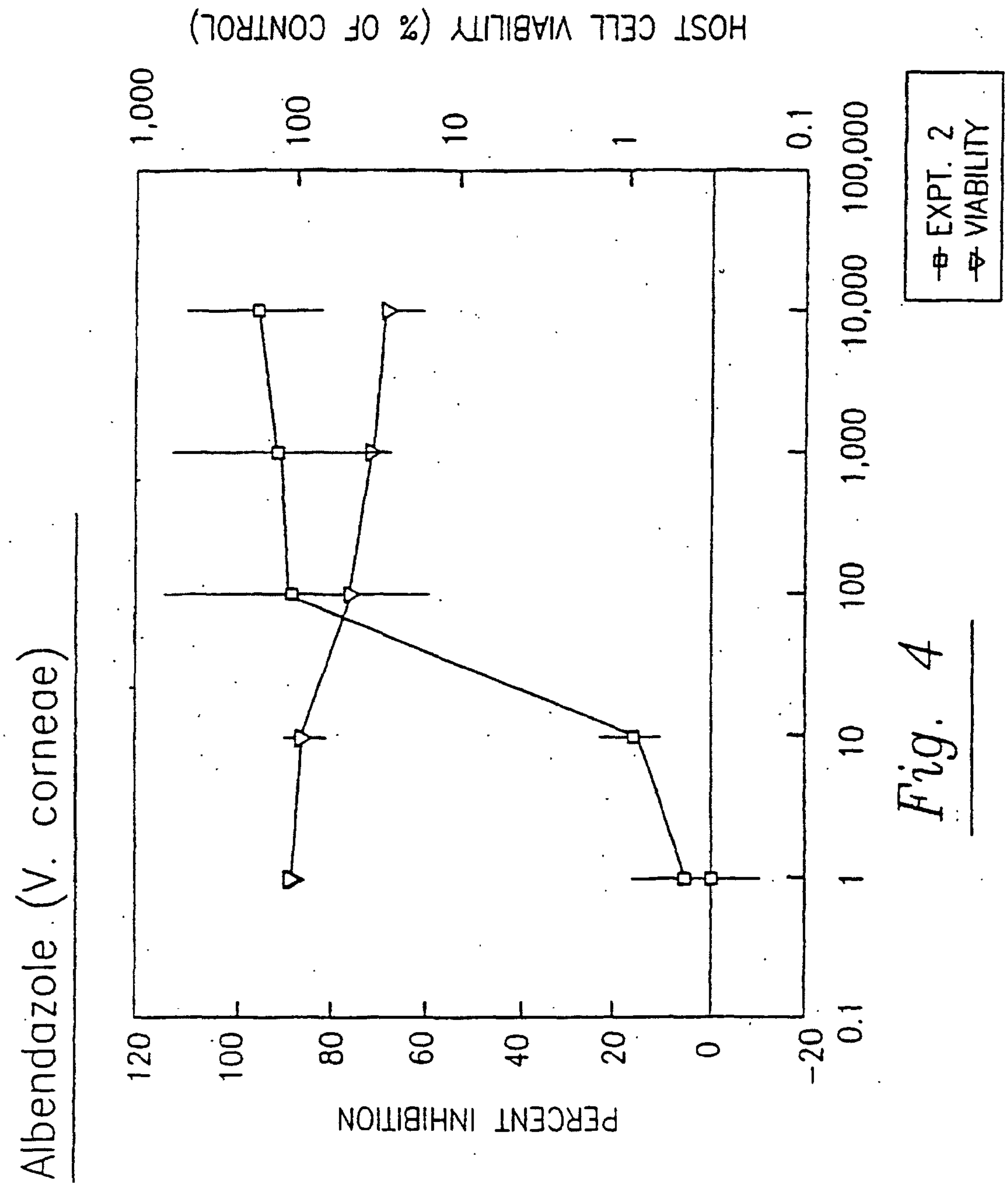
Albendazole (V. corneae)
PERCENT INHIBITION
120
100
80
60
40
20
0
-20
0.1
1
10
100
1,000
10,000
100,000
HOST CELL VIABILITY (% OF CONTROL)
1,000
100
10
1
0.1
EXPT. 2
VIABILITY

*Fig. 4*

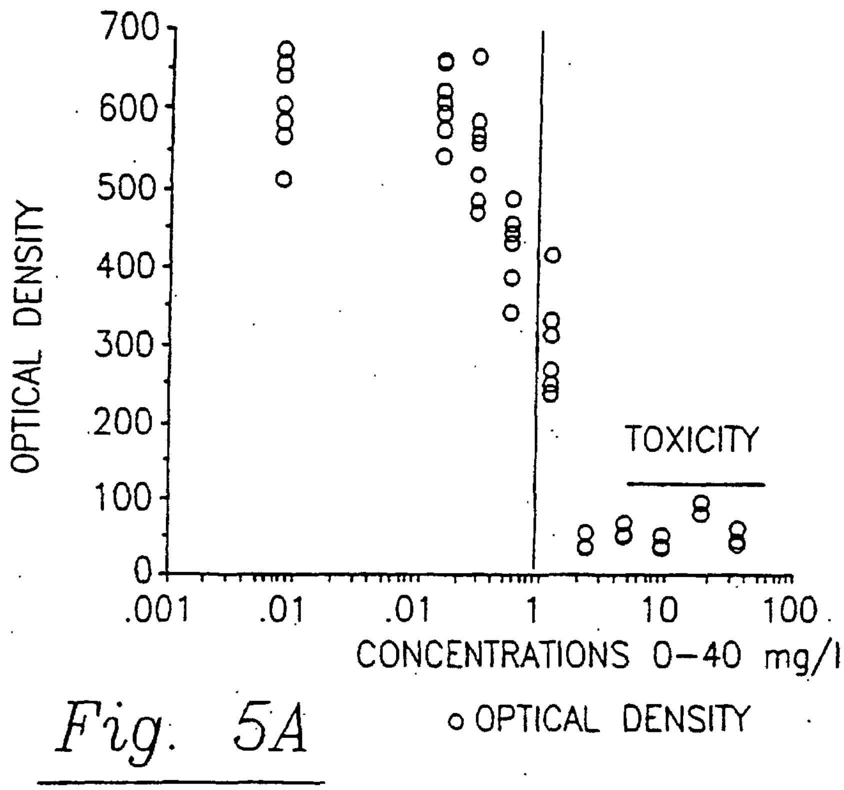
OPTICAL DENSITY
700
600
500
400
300
200
100
0
.001
.01
.01
1
10
100
TOXICITY
CONCENTRATIONS 0-40 mg/l
o OPTICAL DENSITY

*Fig. 5A*

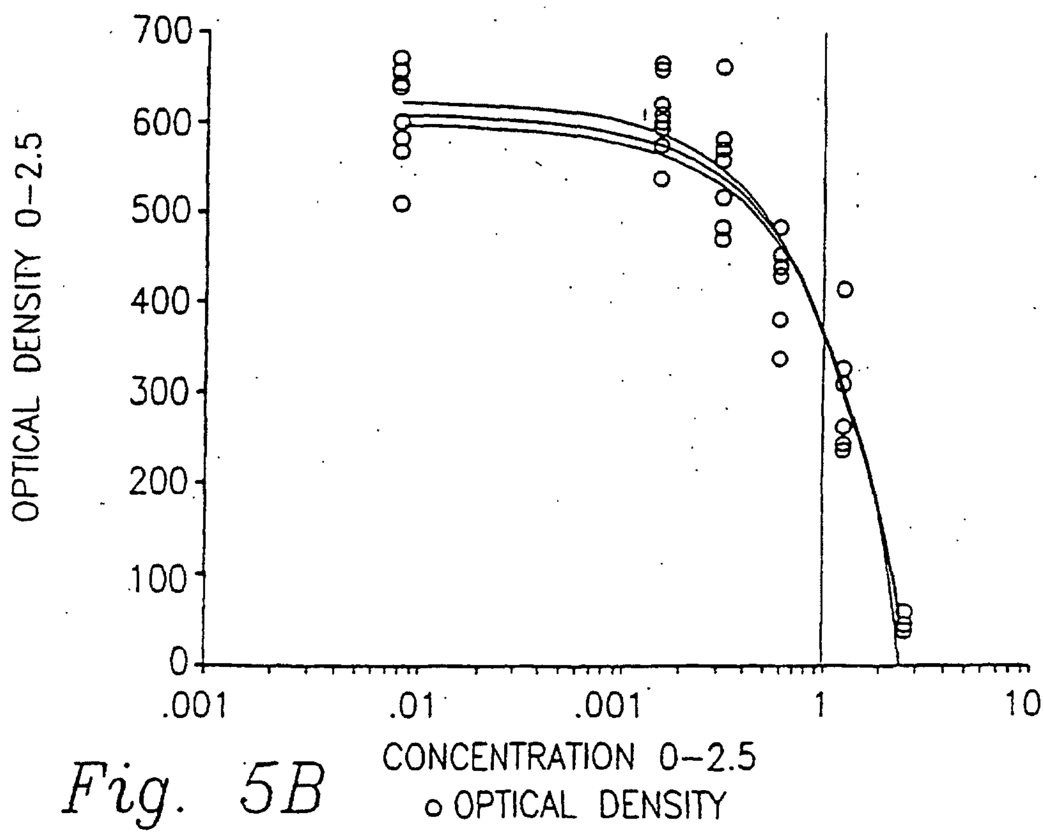
OPTICAL DENSITY 0-2.5
700
600
500
400
300
200
100
0
.001
.01
.001
1
10
CONCENTRATION 0-2.5
o OPTICAL DENSITY

*Fig. 5B*

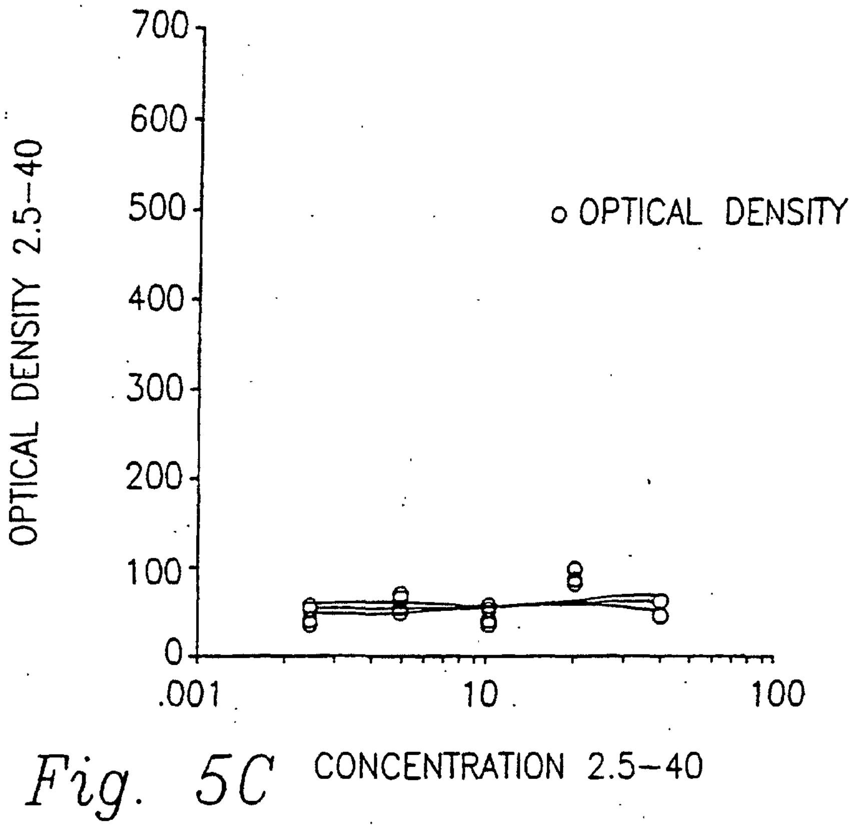
700
600
500
400
300
200
100
0
OPTICAL DENSITY 2.5–40
o OPTICAL DENSITY
.001
10
100
CONCENTRATION 2.5–40

*Fig. 5C*

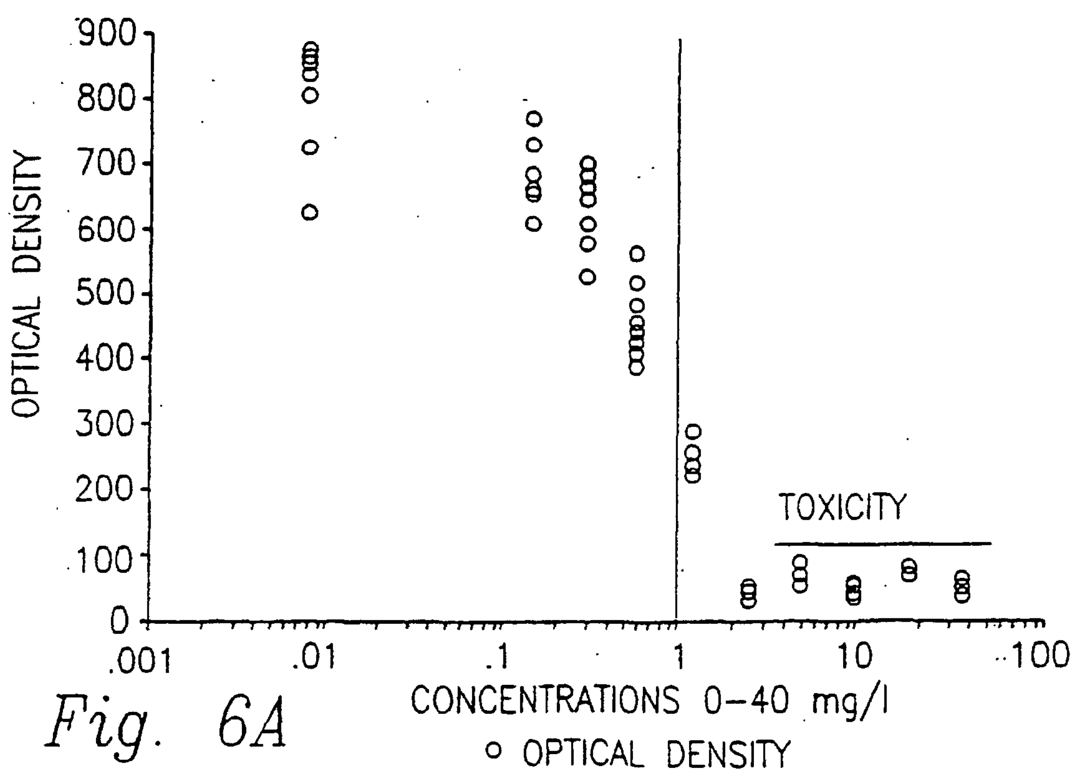
900
800
700
600
500
400
300
200
100
0
OPTICAL DENSITY
TOXICITY
.001
.01
.1
1
10
100
CONCENTRATIONS 0–40 mg/l
o OPTICAL DENSITY

*Fig. 6A*

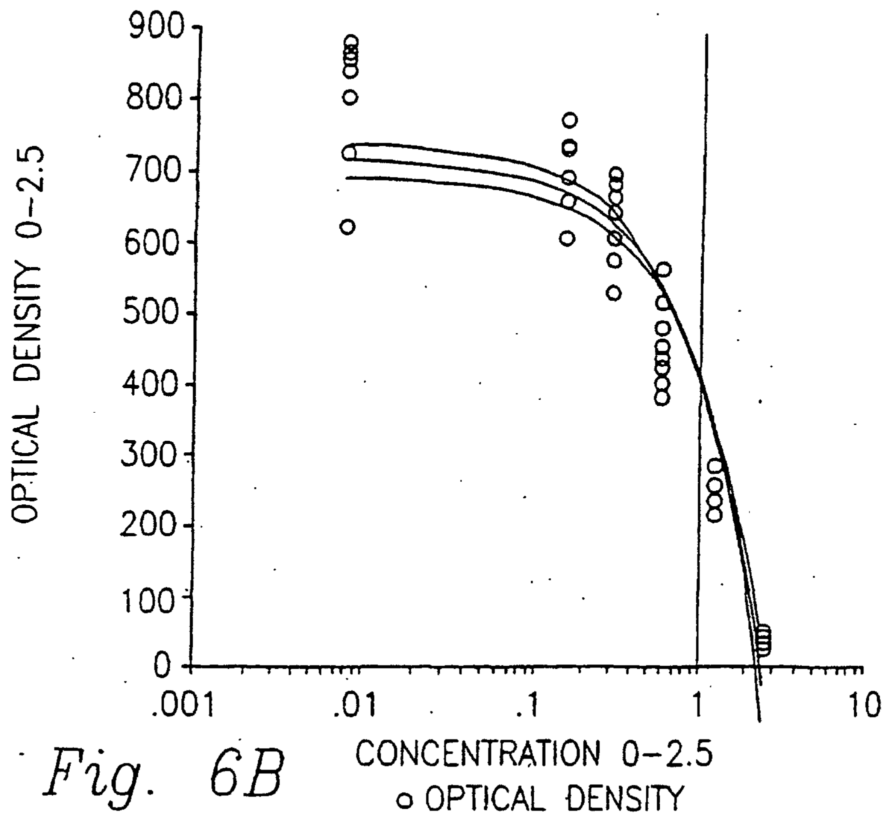
900
800
700
600
500
400
300
200
100
0
OPTICAL DENSITY 0-2.5
.001
.01
.1
1
10
CONCENTRATION 0-2.5
o OPTICAL DENSITY

*Fig. 6B*

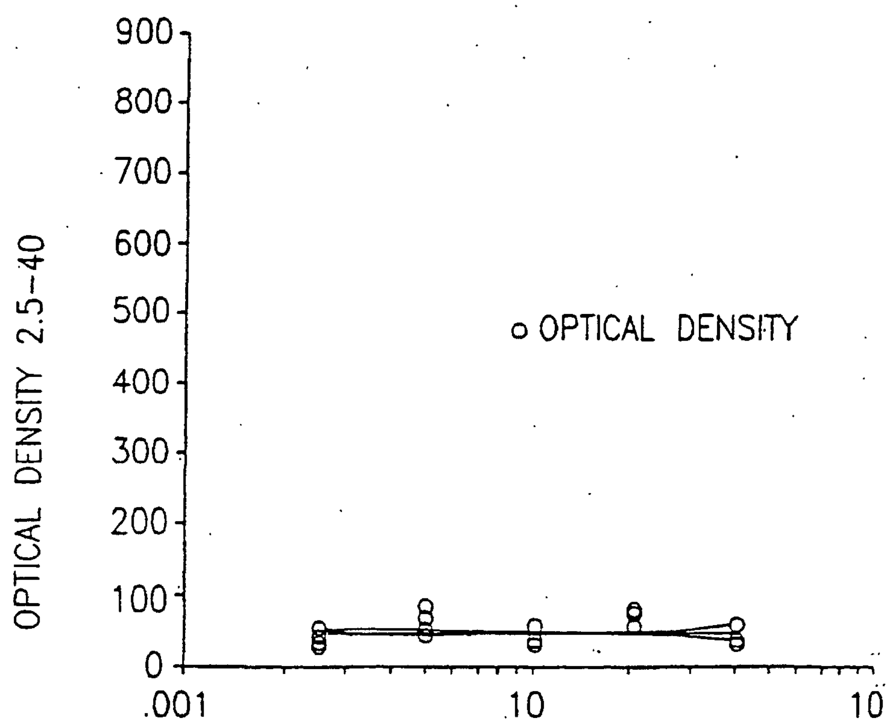
900
800
700
600
500
400
300
200
100
0
OPTICAL DENSITY 2.5-40
o OPTICAL DENSITY
.001
10
10
CONCENTRATION 2.5-40

*Fig. 6C*

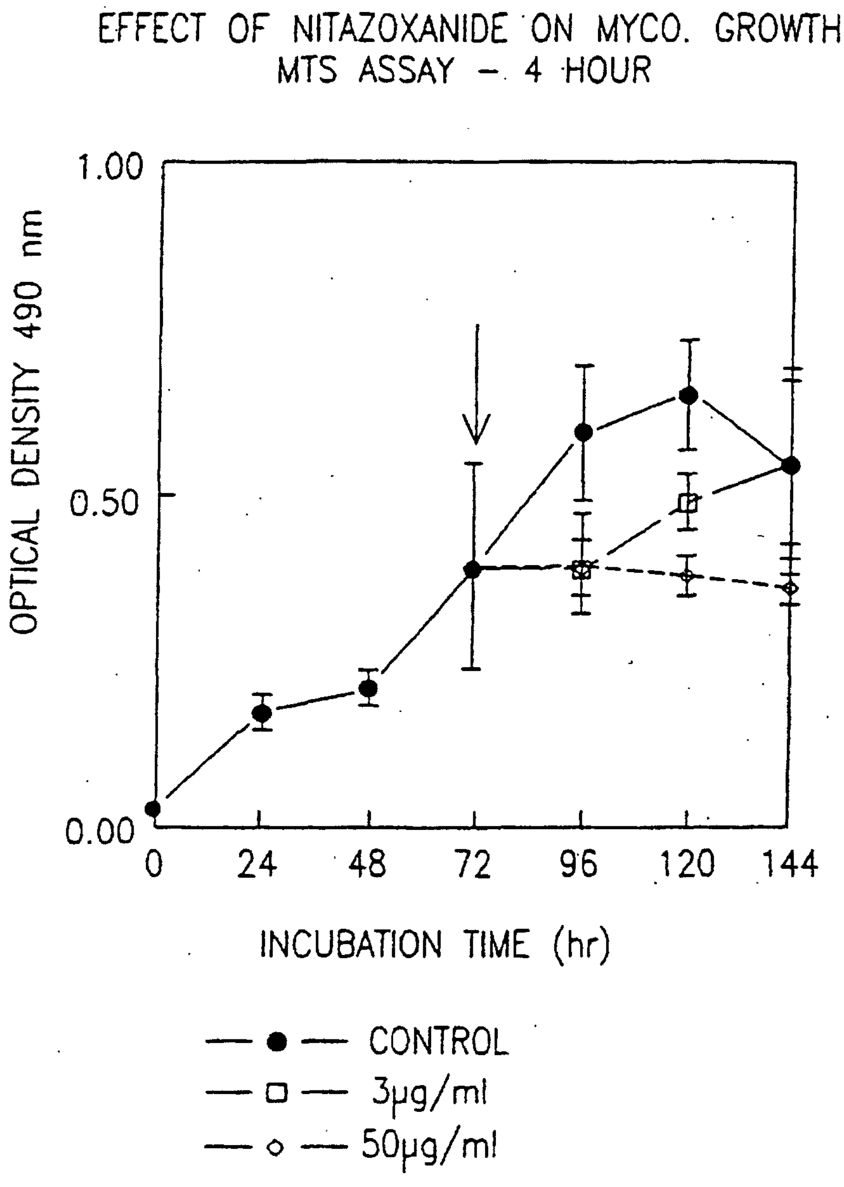
EFFECT OF NITAZOXANIDE ON MYCO. GROWTH
MTS ASSAY - 4 HOUR
OPTICAL DENSITY 490 nm
1.00
0.50
0.00
0
24
48
72
96
120
144
INCUBATION TIME (hr)
CONTROL
3μg/ml
50μg/ml

*Fig. 7*

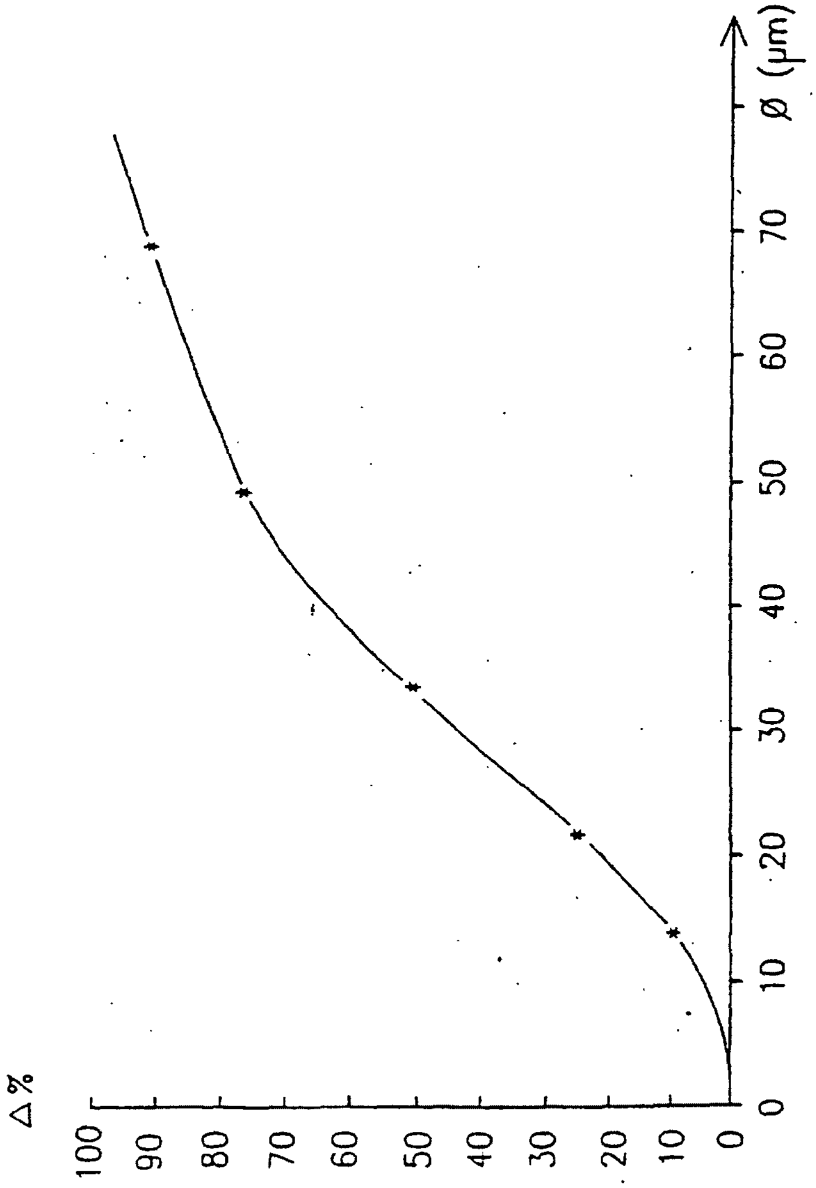


*Fig. 8*